# EUROPEAN PATENT APPLICATION

(11) **EP 4 435 004 A1**
(43) Date of publication of application: **25.09.2024**
(21) Application number: 23191749.3
(22) Date of filing: 16.08.2023
(51) Int. Cl.: C07K 16/24, C07K 16/46, A61K 39/395, A61P 11/06

(54) **ASTHMA TREATMENT BY BLOCKING IL-13 AND TSLP**

(30) Priority: 24.03.2023 EP 23305412
(71) Applicant: Sanofi, 75017 Paris (FR)
(72) Inventor: The designation of the inventor has not yet been filed
(74) Representative: Hoffmann Eitle

(57) **Abstract**

The present invention provides compounds for use in the treatment of pulmonary diseases, in particular asthma, wherein said compounds binds bind the cytokines IL-13 and TSLP. The treatments according to the present invention can lower the level of fractional exhaled nitric oxide (FeND) at least by 18 ppb compared to placebo. The treatments of the present invention can also reduce eosinophil count, increase FEV1 and/or reduce airway inflammation.

## Description

### 1 Field of the present invention

The present invention relates to the treatment of asthma by administering a compound with binds to IL-13 and TSLP. Blocking those two cytokines shows remarkable effect such as reducing the level of fractional exhaled nitric oxide (FeNO) by at least 18 ppb compared to a control such a placebo and/or reducing the eosinophil count in blood by at least 30% compared to a control such a placebo. The treatment also reduces airway inflammation and reduces obstruction of the small airways.

### 2 Background

While necessary for host-defense, unrestrained immune responses can lead to a range of inflammatory diseases such as asthma, atopic dermatitis, and rheumatoid arthritis. A cascade of immune responses mediated by the innate and adaptive arms of the immune system (e.g., antigen recognition, antigen processing, antigen presentation, cytokine production, antibody production, target cell killing) drive the initiation and propagation of a range of immunological diseases. Inflammatory diseases are often chronic and can even be life-threatening. Allergic and atopic diseases such as asthma are often driven predominantly by type 2 immune responses and characterized by salient features of type 2 immunity such as high IgE production and eosinophilia.

Thymic stromal lymphopoietin (TSLP) and Interleukin-13 (IL-13) are soluble cytokine targets produced by stromal and/or immune cells (Ziegler & Artis, Nat Rev Immunol (2010) 11:289, Gieseck III et al., Nat Rev Immunol (2018) 18:62). Human TSLP and IL-13 drive distinct, overlapping and synergistic aspects of immunity and autoimmunity, such as type 2 inflammation.

The signaling of TSLP begins through a heterodimeric receptor complex composed of the thymic stromal lymphopoietin receptor (TSLPR) and the IL-7R alpha chain (IL-7Rα). Similarly, IL-13 signaling starts by binding to a heterodimeric receptor complex consisting of alpha IL-4 receptor (IL-4Rα) and alpha Interleukin-13 receptor (IL-13R1α). The high affinity of IL-13 to the IL-13R1 leads to their complex formation which further increase the probability of a heterodimer formation to IL-4Rα.

TSLP drives the maturation of dendritic cells, development and proliferation of mast cells, as well as activating other immune cells such as basophils and innate lymphoid cells (ILC2). Similarly, IL-13 exerts a range of immunopathologies such as epithelial barrier disruption, mucus production from mucosal-epithelial surfaces, airway remodeling as well as the induction of eosinophil recruiting chemokines such as eotaxin. These mechanisms are central to the initiation and propagation of the type 2 inflammatory response and are central to the development of a range of immunopathologies in diseases such as atopic dermatitis and asthma.

Currently, patients with moderate/severe asthma are inadequately responding to presently available standard of care treatments, including biological such as anti-IL4Rα monoclonal antibody Dupixent (dupilumab; marketed), anti-IL5s (marketed), anti-IgE monoclonal antibody Xolair (omalizumab; marketed), in particular in asthma patients with a low eosinophilic phenotype. Although antagonistic monoclonal antibodies against TSLP (tezepelumab, marketed) and IL-13 (lebrikizumab) exist, there is no marketed compounds that target both TSLP and IL-13. Dual targeting of TSLP and IL-13 with a single compound may have the potential to confer efficacy in both low eosinophilic and high eosinophilic asthma, with the potential to confer efficacy in sub-populations within these indications where a single monospecific agent therapy may not be fully efficacious.

Targeting multiple disease factors may be achieved for example by co-administration or combinatorial use of two separate biologicals, e.g., antibodies binding to different therapeutic targets. However, co-administration or combinatorial use of separate biologicals can be challenging, both from a practical and a commercial point of view. For example, two injections of separate products result in a more inconvenient and more painful treatment regime to the patients which may negatively affect compliance. With regard to a single injection of two separate products, it can be difficult or impossible to provide formulations that allow for acceptable viscosity at the required concentrations and suitable stability of both products. Additionally, co-administration and co-formulation requires production of two separate drugs which can increase overall costs. Bispecific antibodies that are able to bind to two different antigens have been suggested as one strategy for addressing such limitations associated with co-administration or combinatorial use of separate biologicals, such as antibodies.

Those factors hamper the development of multispecific asthma therapies. Thus, there is a high need of alternative asthma treatments which target multiple pathological pathways. The present invention meets this need by providing a compound that binds both TSLP and IL-13 for use in treating a pulmonary disease, e.g. asthma. The present invention shows for the first time that simultaneous targeting of TSLP and IL-13 is highly effective in asthma patients. In particular, the present invention shows that treatment with a compound which blocks TSLP and IL-13 leads to improvement of specific clinical parameters, like FeNO levels, to a much higher extent than existing asthma medication.

### 3 Summary of the present invention

The present invention shows for the first time the strong effect of simultaneously blocking TSLP and IL-13 in human subjects suffering from a pulmonary disease, like asthma. In particular, this effect materializes in a reduction of FeNO level by at least 18 ppb compared to baseline FeNO level or to placebo and/or a reduction of eosinophil count by at least 30% compared to baseline eosinophil count or to placebo and/or an increase of the forced expiratory volume in one second (FEV1) by at least 0.07 L compared to baseline FEV1 or to placebo. The present invention shows that simultaneously blocking TSLP and IL-13 in human subjects reduces airway inflammation and improves lung function.

In another aspect, the present invention relates to a preventive aspect. As the present invention provides ways to reduce FeNO level to a larger extent than known treatments, this opens the possibility of preventively treat patients with elevated FeNO level to prevent a loss of lung function before it even occurs. This preventive treatment can be administered to asthma patients and subjects having the risk of developing asthma as well as patients with other pulmonary diseases and subjects having the risk of developing other pulmonary diseases. Thus, the present invention relates to compounds for use in reducing the FeNO level in a subject wherein the reduction of FeNO level prevents a loss of lung function.

The present invention provides the following exemplary embodiments:
A compound that binds IL-13 and TSLP for use in the treatment of a pulmonary disease, wherein said treatment reduces the level of fractional exhaled nitric oxide (FeNO) by at least 18 ppb compared to a control.

The compound of use according to the previous embodiment, wherein the treatment reduces the level of FeNO by at least 20 ppb, at least 30 ppb, or by at least 40 ppb compared to a control.

The compound for use according to any of the previous embodiments, wherein the pulmonary disease is asthma.

The compound for use according to the previous embodiment, wherein the asthma is high eosinophilic asthma.

The compound for use according to the previous but one embodiment, wherein the asthma is low eosinophilic asthma.

The compound for use according to any of the previous embodiments, wherein the control is baseline or wherein the control is placebo.

The compound for use according to any of the previous embodiments, wherein said reduction of FeNO level occurs within 4 weeks after the administration of the compound, wherein optionally said reduction of FeNO level occurs within 2 weeks after the administration of the compound, wherein optionally said reduction of FeNO level occurs within 1 week after the administration of the compound.

The compound for use according to any of the previous embodiments, wherein the compound that binds IL-13 and TSLP is a polypeptide, such as an antibody or an antibody fragment.

The compound for use according to the previous embodiment, wherein the polypeptide comprises or consists of at least four ISVDs, wherein two ISVDs specifically bind IL-13 and two ISVDs specifically bind TSLP, wherein each of said at least four ISVDs comprises three complementarity determining regions (CDR1 to CDR3, respectively), wherein the at least four ISVDs are optionally linked via one or more peptidic linkers, and wherein:
a first ISVD comprises:
   a CDR1 that is the amino acid sequence of SEQ ID NO: 7, a CDR2 that is the amino acid sequence of SEQ ID NO: 12 and a CDR3 that is the amino acid sequence of SEQ ID NO: 17,
a second ISVD comprises:
   a CDR1 that is the amino acid sequence of SEQ ID NO: 8, a CDR2 that is the amino acid sequence of SEQ ID NO: 13 and a CDR3 that is the amino acid sequence of SEQ ID NO: 18,
a third ISVD comprises:
   a CDR1 that is the amino acid sequence of SEQ ID NO: 9, a CDR2 that is the amino acid sequence of SEQ ID NO: 14 and a CDR3 that is the amino acid sequence of SEQ ID NO: 19, and
a fourth ISVD comprises:
   a CDR1 that is the amino acid sequence of SEQ ID NO: 11, a CDR2 that is the amino acid sequence of SEQ ID NO: 16 and a CDR3 that is the amino acid sequence of SEQ ID NO: 21.

The compound for use according to one of the two previous embodiments, wherein the polypeptide comprises or consists of the amino acid sequence of SEQ ID NO: 1.

A compound that binds IL-13 and TSLP for use in the treatment of a pulmonary disease, wherein said treatment reduces the eosinophil count in blood by at least 30% compared to a control.

The compound of use according to the previous embodiment, wherein said reduction of the eosinophil count occurs within 4 weeks after the administration of the compound, wherein optionally said reduction of the eosinophil count occurs within 2 weeks after the administration of the compound, wherein optionally said reduction of the eosinophil count occurs within 1 week after the administration of the compound.

A compound that binds IL-13 and TSLP for use in the treatment of a pulmonary disease, wherein said treatment reduces airway inflammation.

The compound of use according to the previous embodiment, wherein said reduction of airway inflammation is characterized by a reduction of FeNO by at least 18 ppb, a reduction of eosinophil count by at least 30% and/or an increase of the forced expiratory volume in one second (FEV1) by at least 0.07 L compared to a control.

The compound of use according to the previous embodiment, wherein said reduction of airway inflammation occurs within 4 weeks after the administration of the compound, wherein optionally said reduction of airway inflammation occurs within 2 weeks after the administration of the compound, wherein optionally said reduction of airway inflammation occurs within 1 week after the administration of the compound.

In another aspect, the present invention provides the following exemplary embodiments: A compound which binds TSLP and/or IL-13 for use in reducing the FeNO level in a subject, wherein the reduction of FeNO level prevents a loss of lung function.

The compound for use according to the previous embodiment, wherein said reduction of FeNO level is a reduction by at least 18 ppb.

The compound for use according to any of the two previous embodiments, wherein said reduction of FeNO level is a reduction to a level of below 25 ppb.

The compound for use according to any of the three previous embodiments, wherein the subject has a baseline FeNO level of at least 50 ppb, at least 35 ppb, or at least 25 ppb.

The compound for use according to any of the four previous embodiments, wherein the compound binds TSLP and IL-13.

The compound for use according to any of the five previous embodiments, wherein said a loss of lung function is linked to asthma.

### 4 Brief description of the drawings

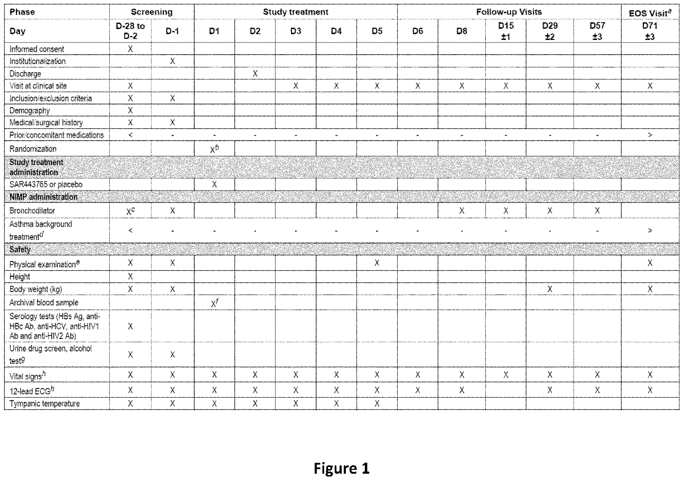

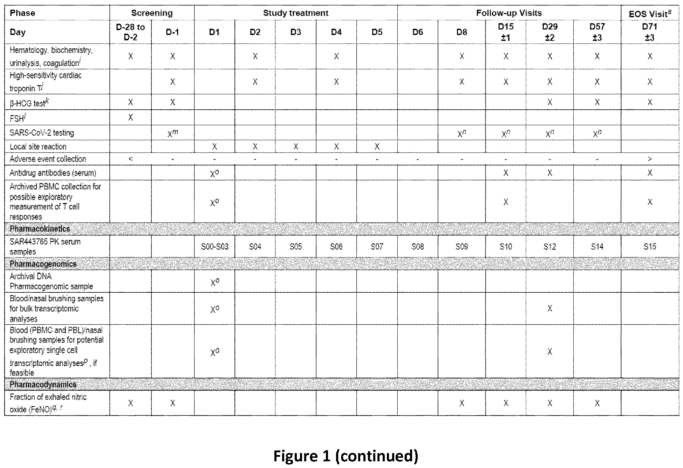

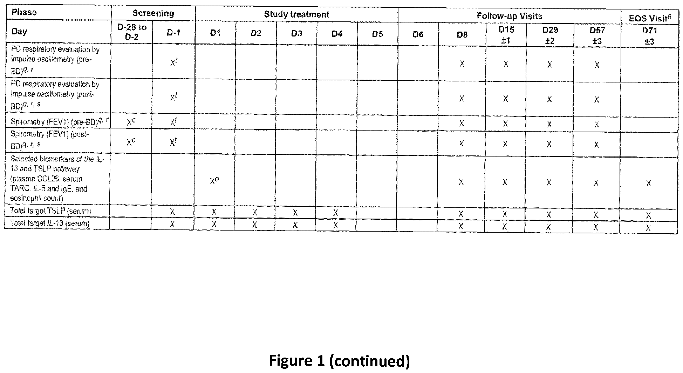

**Figure 1****:** Schedule of the clinical trial of example 1 (PDY16622). The schedule indicates the treatments/measurements which were performed at the indicated time points ("D" for "day") of the clinical trial.

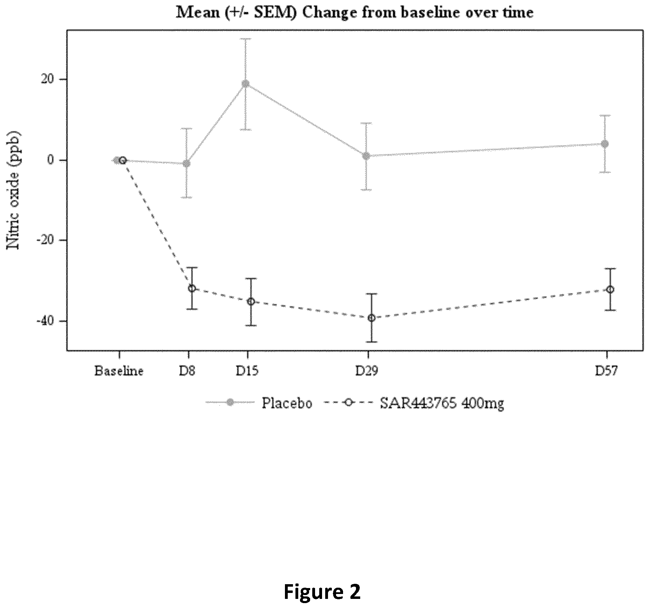

**Figure 2****:** FeNO measurements of the clinical trial of example 1 (PDY16622). Shown is the change in level of fractional exhaled nitric oxide (in parts per billion, ppb) from baseline of the SAR443765 group (dashed line) and the placebo group (solid line).

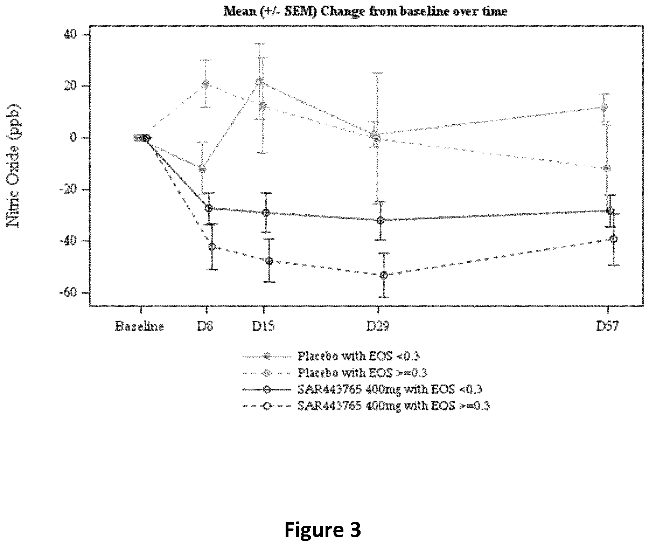

**Figure 3****:** FeNO measurements of the clinical trial of example 1 (PDY16622), analyzed according to eosinophil high/low subgroups. Shown is the change in level of fractional exhaled nitric oxide (in parts per billion, ppb) from baseline of the high eosinophilic SAR443765 group (lower dashed line, dark grey), the low eosinophilic SAR443765 group (lower solid line, dark grey), the high eosinophilic placebo group (upper dashed line, light grey) and the low eosinophilic placebo group (upper solid line, light grey).

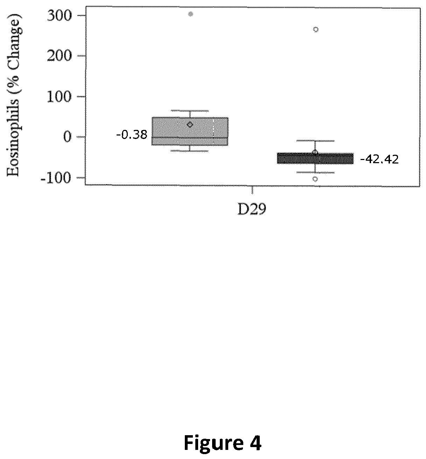

**Figure 4****:** Eosinophil count of the clinical trial of example 1 (PDY16622). Shown is the median change of eosinophil count compared to baseline at D29 (4 weeks) of the SAR443765 group (right) and the placebo group (left).

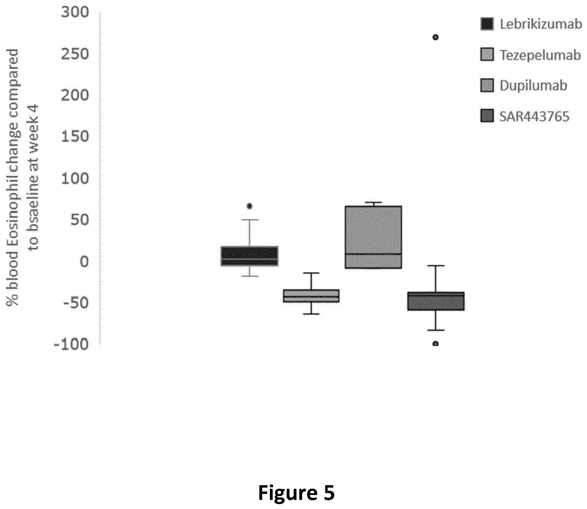

**Figure 5****:** Eosinophil count of clinical trial of example 1 (PDY16622, right bar) compared to three other biologics: lebrikizumab (left bar), tezepelumab (second bar from the left), and dupilumab (second bar from the right) at D29.

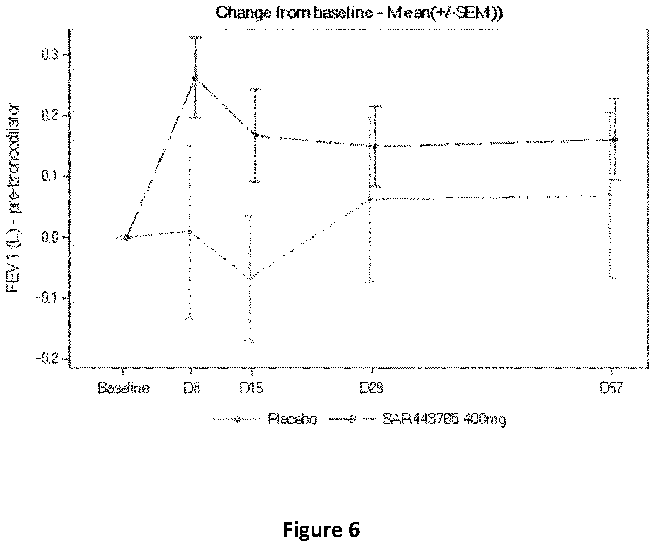

**Figure 6****:** FEV1 measurements of the clinical trial of example 1 (PDY16622). Shown is the change of FEV1 (in liter, compared to baseline) of the SAR443765 group (dashed line) and the placebo group (solid line). All measurements were included.

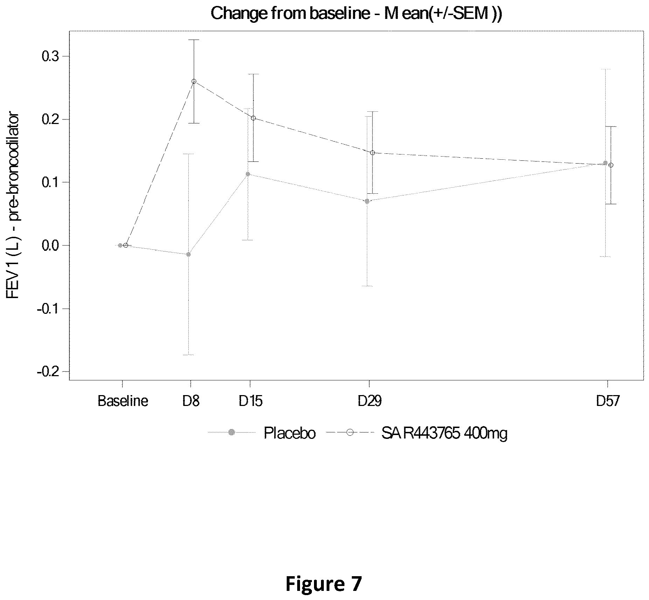

**Figure 7****:** FEV1 measurements of the clinical trial of example 1 (PDY16622). Shown is the change of FEV1 (in liter, compared to baseline) of the SAR443765 group (dashed line) and the placebo group (solid line). Only measurements which fulfilled all quality criteria were included.

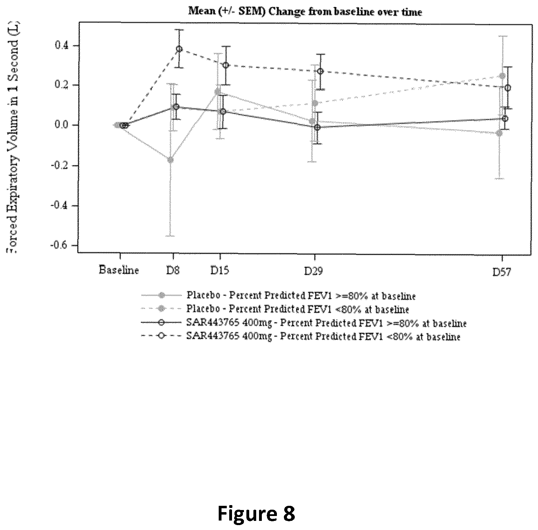

**Figure 8****:** FEV1 measurements of the clinical trial of example 1 (PDY16622). Shown is the change of FEV1 (in liter, compared to baseline) as shown in Fig. 7. The SAR443765 group and the placebo group were each divided into two subpopulations, depending on the baseline percent predicted FEV1 (ppFEV1). The subpopulations are: SAR443765, baseline ppFEV1 ≥ 80% (dark grey solid line); SAR443765, baseline ppFEV1 < 80% (dark grey dashed line); placebo, baseline ppFEV1 ≥ 80% (light grey solid line); placebo, baseline ppFEV1 < 80% (light grey dashed line).

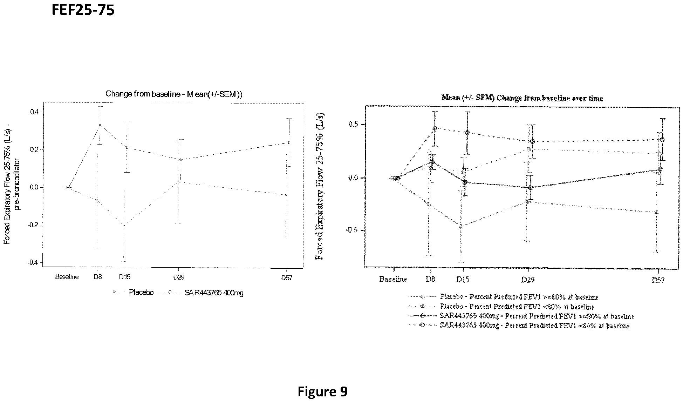

**Figure 9****:** Forced Expiratory Flow 25-75% (FEF25-75) measurements of the clinical trial of example 1 (PDY16622). Shown is the change of FEF25-75 (in liter/s, compared to baseline). The left panel shows the change of FEF25-75 of the SAR443765 group (dashed line) and the placebo group (solid line). In the right panel, the SAR443765 group and the placebo group were each divided into two subpopulations depending on the baseline percent predicted FEV1 (ppFEV1). The subpopulations are: SAR443765, baseline ppFEV1 ≥ 80% (dark grey solid line); SAR443765, baseline ppFEV1 < 80% (dark grey dashed line); placebo, baseline ppFEV1 ≥ 80% (light grey solid line); placebo, baseline ppFEV1 < 80% (light grey dashed line).

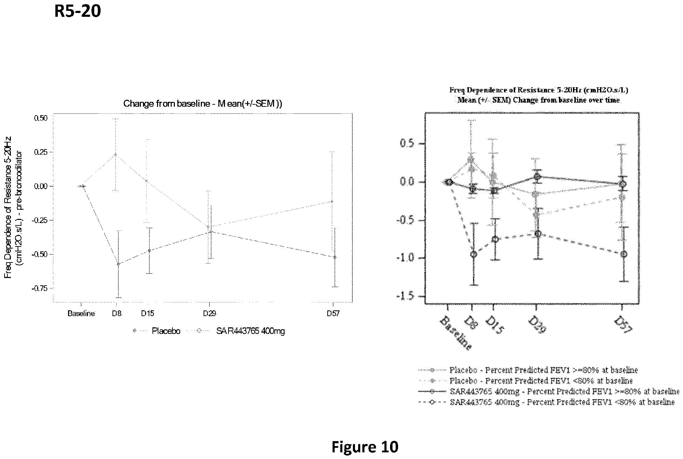

**Figure 10****:** Measurements of the difference of respiratory resistances at 5 Hz and 20 Hz (R5-20) of the clinical trial of example 1 (PDY16622). Shown is the change (in cmH₂O^{∗}s/L, compared to baseline) in the difference between the respiratory resistance at 5 Hz and the respiratory resistance at 20 Hz. The left panel shows the change of R5-20 of the SAR443765 group (dashed line) and the placebo group (solid line). In the right panel, the SAR443765 group and the placebo group were each divided into two subpopulations depending on the baseline percent predicted FEV1 (ppFEV1). The subpopulations are: SAR443765, baseline ppFEV1 ≥ 80% (dark grey solid line); SAR443765, baseline ppFEV1 < 80% (dark grey dashed line); placebo, baseline ppFEV1 ≥ 80% (light grey solid line); placebo, baseline ppFEV1 < 80% (light grey dashed line).

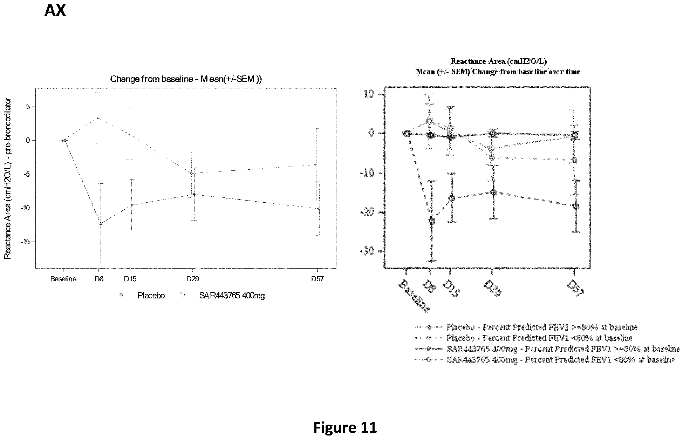

**Figure 11****:** Measurements of the reactance area (AX) of the clinical trial of example 1 (PDY16622). Shown is the change in the reactance area (in cmH₂O/L), compared to baseline). The left panel shows the change of the reactance area of the SAR443765 group (dashed line) and the placebo group (solid line). In the right panel, the SAR443765 group and the placebo group were each divided into two subpopulations depending on the baseline percent predicted FEV1 (ppFEV1). The subpopulations are: SAR443765, baseline ppFEV1 ≥ 80% (dark grey solid line); SAR443765, baseline ppFEV1 < 80% (dark grey dashed line); placebo, baseline ppFEV1 ≥ 80% (light grey solid line); placebo, baseline ppFEV1 < 80% (light grey dashed line).

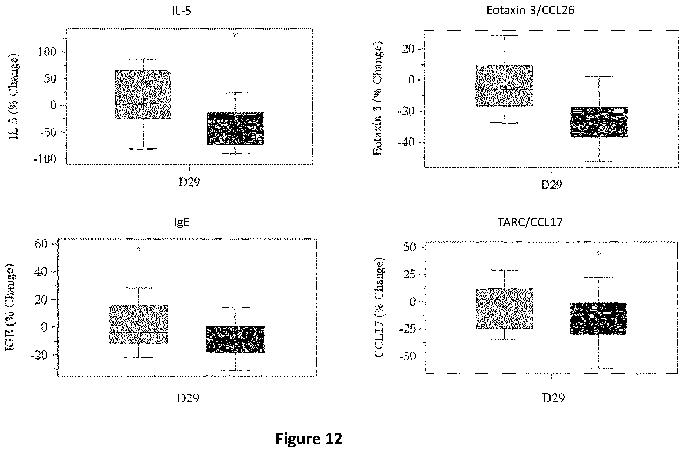

**Figure 12****:** Further biomarker measurements of the clinical trial of example 1 (PDY16622). Shown is the change of IL-5 level in serum (upper left panel); CCL26 (eotaxin-3) level in plasma (upper right panel), IgE level in serum (lower left panel), and TARC (CCL17) level in serum (lower right panel) and compared to baseline. Each panel shows the results for placebo (left) and SAR443765 (right).

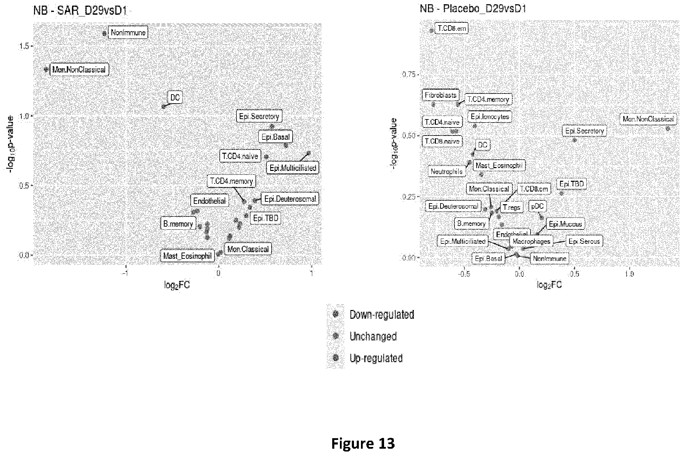

**Figure 13****:** Change in cell types in nasal brushing samples of the clinical trial of example 1 (PDY16622). Shown is the change between samples from D1 (baseline, before SAR443765 or placebo administration) and D29. The change in cell type proportions of each cell type (indicated as log2 of fold-change (FC)) on the x-axis is plotted against the p-value for this change (indicates as -log10 of the p-value) on the y-axis.

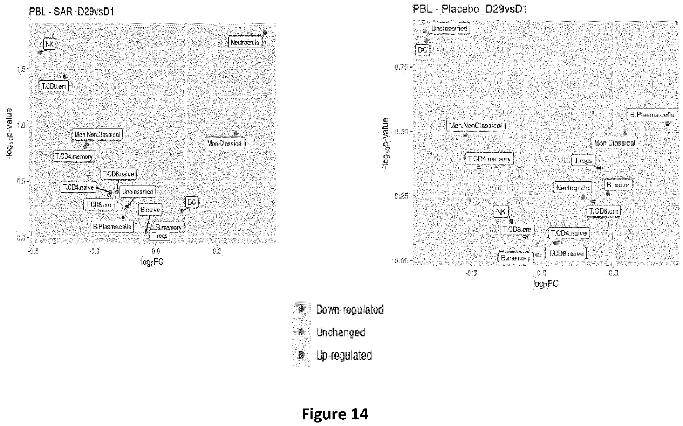

**Figure 14****:** Change in cell types in peripheral blood leukocytes (PBL) samples of the clinical trial of example 1 (PDY16622). Shown is the change between samples from D1 (baseline, before SAR443765 or placebo administration) and D29. The change in cell type proportions of each cell type (indicated as log2 of fold-change (FC)) on the x-axis is plotted against the p-value for this change (indicates as -log10 of the p-value) on the y-axis.

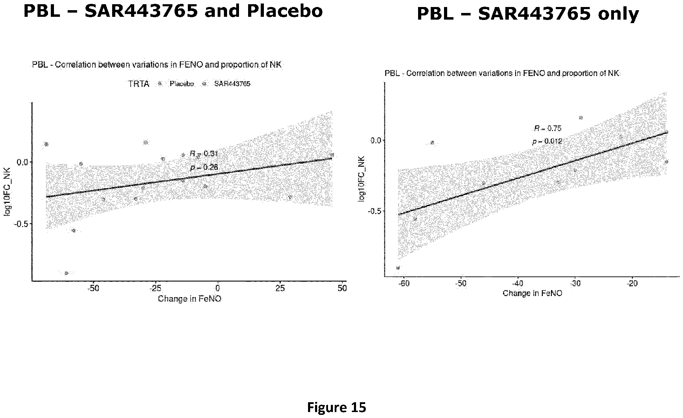

**Figure 15****:** Correlation of the change in NK cell proportion (in PBL samples) and change in FeNO level in the clinical trial of example 1 (PDY16622). Shown is the change between samples/measurements from D1 (baseline, before SAR443765 or placebo administration) and D29. The change in FeNO on the x-axis is plotted against the change in cell type proportion (D29-D1) of NK cells (indicated as log10 of fold-change (FC)) on the y-axis.
**Figure 16****:** Expression of CCL26 on D1 and D29 in nasal brushing samples of the clinical trial of example 1 (PDY16622). In the SAR443765 group, there is a significant difference in CCL26 expression between D1 and D29 for the cell types Epithelial Basal, Epithelial Multiciliate, and Epithelial Secretory.

### 5 Detailed description of the present invention

In a first aspect, the present invention relates to a compound which blocks TSLP and IL-13 for the use in treating a pulmonary disease, e.g., asthma. This treatment is characterized by the improvement of certain biomarkers, in particular a reduction in FeNO level and a reduction of the eosinophil count.

In another aspect, the present invention relates to compounds for use in reducing the FeNO level in a subject wherein the reduction of FeNO level prevents a loss of lung function. This loss of lung function can be linked to asthma or to another pulmonary disease.

### 5.1 Definitions

Unless otherwise stated below, all terms used in this application, including the specification and claims, have the meaning usually given to them in the respective scientific field.

As used in the specification and the appended claims, the indefinite articles "a" and "an" and the definite article "the" include plural as well as singular referents unless the context clearly dictates otherwise.

All indications of time spans in this application count the day of drug administration as day 1 ("D1"). This means that the day following drug administration is counted as day 2 ("D2"), so that, e.g., a measurement which occurs 24 h after drug administration is on D2 and a measurement which occurs 72 h after drug administration is on D4. This means also that a time span of one week ends on D8, a time span of 2 weeks ends on D15, a time span of three weeks ends on D22, a time span of 4 weeks ends on D29, a time span of 8 weeks ends on D57, and a time span of 10 weeks ends on D71. In the present disclosure, "day" is often abbreviated "D". The terms "day" and "D" are used interchangeably in this closure.

A pulmonary disease is a disease of the lung. An example for a pulmonary disease is asthma.

An Inflammatory disease is a disease which is characterized by auto-inflammation. There are inflammatory diseases of the lung. An example for such a pulmonary inflammatory disease is asthma.

Asthma is a pulmonary disease which is characterized by long-term airway inflammation. Asthma comprises airflow obstruction and triggered bronchospasms. Symptoms often include episodes of wheezing, coughing, chest tightness, and shortness of breath. Asthma includes to allergic, non-allergic, Th2 high, Th2 low, high eosinophilic, and low eosinophilic asthma.

High eosinophilic asthma, as used herein, is asthma wherein the patient shows an eosinophil count of more than or equal 0.3 ^{∗}10⁹ cells/L.

Low eosinophilic asthma, as used herein, is asthma wherein the patient shows an eosinophil count of less than 0.3 ^{∗}10⁹ cells/L.

Airway inflammation is an inflammation which is located in the respiratory tract. Airway inflammation can be evaluated directly, e.g., by an induced sputum analysis, bronchial wash, bronchial biopsy or exhaled volatile markers such as FeNO or indirectly, e.g. by increased eosinophil count in blood. Airway inflammation is characterized by an elevated FeNO level, an elevated eosinophil count in blood and decreased lung function (e.g. decrease FEV1).

Type 2 inflammation is an immune response which is characterized by the activation of type 2 T helper cells and/or type 2 innate lymphoid cells. The immune response of a type 2 inflammation is characterized by the release of alarmins (IL-23, IL-33, TSLP) which leads to the activation of type 2 T helper cells and/or type 2 innate lymphoid cells. Those cells secrete IL-4, IL-5 and IL-13, which promotes isotype switching to IgE in B cells and eosinophil recruitment. A type 2 inflammation can be useful for the defense of the body against helminths but is involved in a variety of autoinflammatory diseases. The airway inflammation in asthma patients is often a type 2 inflammation.

Fractional exhaled nitric oxide (FeNO) is the fraction of nitric oxide (NO) in the exhaled air. It is measured in parts per billion (ppb). An elevated FeNO level is a sign of airway inflammation. When the present application deals with "reduction of FeNO level compared to placebo", this refers to the means of the individual changes from baseline of the FeNO level of the compound group (in ppb) and the placebo group (in ppb) at a certain time point. The difference between those two values (in ppb) is the "reduction of FeNO level compared to placebo".

Managing elevated FeNO level, as used herein, means administering a treatment which reduces elevated FeNO level which may reduce airway inflammation and/or prevent the exacerbation of airway inflammation.

Managing airway inflammation, as used herein, means administering a treatment which reduces airway inflammation whereby symptoms associated with airway inflammation are reduced and/or the exacerbation of symptoms associated with airway inflammation is prevented.

Eosinophil count, as used herein, is the number of eosinophilic granulocytes. In the treatments according to the present invention, the eosinophil count is measured in whole blood. Methods to measure the eosinophil count are known in the art, e.g. flow cytometry or counting under a microscope after H&E staining. The eosinophil count is measured in cells / L. When the present application deals with "reduction of eosinophil count compared to placebo", this means that, at a certain time point after start of treatment, the change from baseline of the eosinophil count of the compound group (in cells / L or %) and the change from baseline of the eosinophil count of the placebo group (in cells / L or %) are measured. The difference between those two values (in cells / L or %) is the "reduction of eosinophil count compared to placebo".

The forced expiratory volume in one second (FEV1) is the volume that has been exhaled at the end of the first second of forced expiration after maximal inspiration. FEV1 can be measured by spirometry. When the present application deals with "increase of FEV1 compared to placebo", this means that, at a certain time point after start of treatment, the change from baseline of FEV1 of the compound group (in L) and the change from baseline of the FEV1 of placebo group (in L) are measured. The difference between those two values (in L) is the "increase of FEV1 compared to placebo".

Placebo, as used herein, is a treatment, or a substance used in such a treatment, which does not include a pharmacologically active compound. In clinical trials, a placebo can be administered to a fraction of the participants to generate a control group for the fraction of participants who receive the pharmacologically active compound to be tested. To serve as a proper control, the treatment with placebo is identical to the treatment it is compared with, the only exception being that a placebo (e.g. an inert pill like a sugar pill) and not the pharmacologically active compound is administered.

In the present invention, the change of a biomarker (e.g. FeNO, eosinophil count) is determined compared to a "control". The control is either the "baseline" of the patients, i.e. the biomarker level of the patients before the administration of the compound of the invention; or "placebo", i.e. the biomarker level after administration of a placebo instead of the compound of the invention. When the change of the biomarker level is determined compared to baseline, this means that the difference between the baseline biomarker level and the biomarker level at a certain time point is calculated for each patient who was treated with the compound and the mean or median of those differences is indicated as change of the biomarker level compared to baseline. When the change of the biomarker level is determined compared to placebo, this means that the change compared to baseline is determined for a patient group treated with the compound (as explained in the previous sentence) and the same value is determined for a patient group treated with placebo; the difference between those two values is indicated as change of the biomarker level compared to placebo. This means that the change of the biomarker level compared to placebo equals the "change of the biomarker level compared to baseline" minus the change of the biomarker level of a placebo group.

Small airways refers to those airways which have a diameter of equal to or less than 2 mm, as commonly defined in the literature (see e.g. McNulty and Usmani, Eur Clin Respir J, 2014 and Stockley et al., Int J Chron Obstruct Pulmon Dis. 2017; 12: 2343-2353).

Large airways refers to those airways which have a diameter of more than 2 mm.

The participants of the clinical trial of example 1 (PDY16622) consisted of asthma patients (see below in example 1). Thus, the terms the terms "participant" and "patient" are used synonymously throughout this application.

### 5.2 Treatment of the present invention

The present invention provides compounds that bind IL-13 and TSLP for use in treating a pulmonary disease. Those treatments are characterized by improvements in biomarkers and/or physiological features such as a reduction of the level of FeNO, a reduction of the eosinophil count in blood, an increase of the FEV1, and/or a reduction of airway inflammation.

The subject of the treatment of the present invention can be any animal, and more specifically a mammal. Among mammals, a distinction can be made between humans and non-human mammals. Non-human animals may be for example companion animals (e.g. dogs, cats), livestock (e.g. bovine, equine, ovine, caprine, or porcine animals), or animals used generally for research purposes and/or for producing antibodies (e.g. mice, rats, rabbits, cats, dogs, goats, sheep, horses, pigs, non-human primates, such as cynomolgus monkeys, or camelids, such as llama or alpaca).

In the context of prophylactic and/or therapeutic purposes, the subject can be any animal, and more specifically any mammal. In one embodiment, the subject is a human subject. Compounds (including polypeptides and nucleic acid molecules) or compositions used in the present invention may be administered to a subject by any suitable route of administration, for example by enteral (such as oral or rectal) or parenteral (such as epicutaneous, sublingual, buccal, nasal, intra-articular, intradermal, intramuscular, intraperitoneal, intravenous, subcutaneous (SC), transdermal, or transmucosal) administration. In one embodiment, substances are administered by parenteral administration, such as intramuscular, subcutaneous or intradermal administration. In one embodiment, subcutaneous administration is used.

An effective amount of a polypeptide, a nucleic acid molecule, or a composition comprising the polypeptide or nucleic acid molecule can be administered to a subject in order to provide the intended treatment results.

One or more doses can be administered. If more than one dose is administered, the doses can be administered in suitable intervals in order to maximize the effect of the polypeptide, composition, nucleic acid molecule or vector.

In some embodiments, the administered amount of the compound that binds TSLP and IL-13 is 400 mg. In some embodiments, the compound that binds TSLP and IL-13 is administered subcutaneously (SC). In some embodiments, only one dose of the compound that binds TSLP and IL-13 is administered.

In some embodiments, the administered amount of the compound that binds TSLP and IL-13 is 400 mg and the compound that binds TSLP and IL-13 is administered subcutaneously (SC). In some embodiments, the administered amount of the compound that binds TSLP and IL-13 is 400 mg and only one dose of the compound that binds TSLP and IL-13 is administered. In some embodiments, the compound that binds TSLP and IL-13 is administered subcutaneously (SC) and only one dose of the compound that binds TSLP and IL-13 is administered. In some embodiments, the administered amount of the compound that binds TSLP and IL-13 is 400 mg, the compound that binds TSLP and IL-13 is administered subcutaneously (SC), and only one dose of the compound that binds TSLP and IL-13 is administered.

### Reduction of the level of FeNO

Reduction of level of FeNO is reflective of reduced airway inflammation and is thus beneficial for patients with pulmonary diseases, e.g., asthma. In some embodiments, the pulmonary disease is an inflammatory disease. In some embodiments, the pulmonary disease is asthma. In some embodiments, the asthma is high eosinophilic asthma. In some embodiments, the asthma is low eosinophilic asthma.

In the present invention, the reduction of the level of FeNO is determined compared to a control. The control is either the "baseline" of the patients, i.e. the FeNO level of the patients before the administration of the compound of the invention; or "placebo", i.e. the FeNO level after administration of a placebo instead of the compound of the invention. When the reduction of FeNO level is determined compared to baseline, this means that the difference between the baseline FeNO level and the FeNO level at a certain time point is calculated for each patient who was treated with the compound and the mean or median of those differences is indicated as reduction of FeNO level compared to baseline. When the reduction of FeNO level is determined compared to placebo, this means that the reduction compared to baseline is determined for a patient group treated with the compound (as explained in the previous sentence) and the same value is determined for a patient group treated with placebo; the difference between those two values is indicated as reduction of FeNO level compared to placebo. This means that the reduction of FeNO level compared to placebo equals the "reduction of FeNO level compared to baseline" minus the change compared to baseline of the FeNO level for a placebo group. In some embodiments, reduction of the level of FeNO is calculated for the mean of a patient group. In some embodiments, reduction of the level of FeNO is calculated for the median of a patient group.

In some embodiments of the present invention, the reduction of the level of FeNO (from baseline) is determined compared to placebo. In some embodiments of the present invention, the level of FeNO is reduced by at least 18 ppb. In some embodiments of the present invention, the level of FeNO is reduced by at least 20 ppb. In some embodiments of the present invention, the level of FeNO is reduced by at least 25 ppb. In some embodiments of the present invention, the level of FeNO is reduced by at least 30 ppb. In some embodiments of the present invention, the level of FeNO is reduced by at least 35 ppb. In some embodiments of the present invention, the level of FeNO is reduced by at least 40 ppb.

The reduction in level of FeNO can be assessed at different time points after the administration of the compound of the present invention. In some embodiments, the reduction in level of FeNO is assessed after 1 week. In some embodiments, the reduction in level of FeNO is assessed after 2 weeks. In some embodiments, the reduction in level of FeNO is assessed after 3 weeks. In some embodiments, the reduction in level of FeNO is assessed after 4 weeks.

The treatment of the present invention can be used to manage elevated FeNO level. In some embodiments, managing FeNO level comprises decreasing the FeNO level to a value of less than 40 ppb. In some embodiments, managing FeNO level comprises decreasing the FeNO level to a value of less than 35. In some embodiments, managing FeNO level comprises decreasing the FeNO level to a value of less than 30. In some embodiments, managing FeNO level comprises decreasing the FeNO level to a value of less than 25. In some embodiments, managing FeNO level comprises decreasing the FeNO level to a value of less than 20.

The FeNO baseline level, i.e. the level before the administration of the compound, can also be a parameter for the treatment of the present invention. In some embodiments, the treatment of the present invention is used to manage elevated FeNO baseline levels of at least 25 ppb. In some embodiments, the treatment of the present invention is used to manage elevated FeNO baseline levels of at least 30 ppb. In some embodiments, the treatment of the present invention is used to manage elevated FeNO baseline levels of at least 40 ppb. In some embodiments, the treatment of the present invention is used to manage elevated FeNO baseline levels of at least 50 ppb. In some embodiments, the treatment of the present invention is used to manage elevated FeNO baseline levels of at least 60 ppb. In some embodiments, the treatment of the present invention is used to manage elevated FeNO baseline levels of at least 70 ppb.

### Reduction of the eosinophil count in blood

Reduction of eosinophil count in blood correlates with reduced airway inflammation, in particular type 2 airway inflammation, and is thus beneficial for patients with pulmonary diseases, e.g., asthma. In some embodiments, the pulmonary disease is an inflammatory disease. In some embodiments, the pulmonary disease is asthma. In some embodiments, the asthma is high eosinophilic asthma. In some embodiments, the asthma is low eosinophilic asthma.

In the present invention, the reduction of the level of eosinophil count is determined compared to a control. The control is either the "baseline" of the patients, i.e. the eosinophil count of the patients before the administration of the compound of the invention; or "placebo", i.e. the eosinophil count after administration of a placebo instead of the compound of the invention. When the reduction of the eosinophil count is determined compared to baseline, this means that the difference between the baseline eosinophil count and the eosinophil count at a certain time point is calculated for each patient who was treated with the compound and the mean or the median of those differences is indicated as reduction of the eosinophil count compared to baseline. When the reduction of the eosinophil count is determined compared to placebo, this means that the reduction compared to baseline is determined for a patient group treated with the compound (as explained in the previous sentence) and the same value is determined for a patient group treated with placebo; the difference between those two values is indicated as reduction of the eosinophil count compared to placebo. This means that the reduction of the eosinophil count compared to placebo equals the "reduction of eosinophil count compared to baseline" minus the change of the eosinophil count of a placebo group. In some embodiments, the reduction of eosinophil count refers to the median. In some embodiments, the reduction of eosinophil count refers to the mean. In some embodiments, the reduction of eosinophil counts refers to the median of the change compared to baseline, expressed in percent.

In some embodiments, the reduction of eosinophil counts refers to the difference between the median change compared to baseline, expressed in percent, for a patient group treated with the compound and the median change compared to baseline, expressed in percent, for the patient group treated with placebo.

In some embodiments of the present invention, the eosinophil count is reduced by at least 30%. In some embodiments of the present invention, the eosinophil count is reduced by at least 30% regarding the median of a patient group. In some embodiments of the present invention, the eosinophil count is reduced by at least 30% regarding the mean of a patient group. In some embodiments of the present invention, the eosinophil count is reduced by at least 35%. In some embodiments of the present invention, the eosinophil count is reduced by at least 35% regarding the median of a patient group. In some embodiments of the present invention, the eosinophil count is reduced by at least 35% regarding the mean of a patient group. In some embodiments of the present invention, the eosinophil count is reduced by at least 40%. In some embodiments of the present invention, the eosinophil count is reduced by at least 40% regarding the median of a patient group. In some embodiments of the present invention, the eosinophil count is reduced by at least 40% regarding the mean of a patient group.

In some embodiments, the reduction of the eosinophil count occurs within 4 weeks after the administration of the compound. In some embodiments, the reduction of the eosinophil count occurs within 2 weeks after the administration of the compound. In some embodiments, the reduction of the eosinophil count occurs within 1 week after the administration of the compound. In some embodiments, the reduction of the eosinophil count occurs within 3 days after the administration of the compound. In some embodiments, the reduction of the eosinophil count occurs within 1 day after the administration of the compound.

In some embodiments, the compound that binds IL-13 and TSLP and which reduces the eosinophil count is a polypeptide, such as an antibody or an antibody fragment. In some embodiments, the compound that binds IL-13 and TSLP and which reduces the eosinophil count is a polypeptide, wherein the polypeptide comprises or consists of at least four ISVDs, wherein two ISVDs specifically bind IL-13 and two ISVDs specifically bind TSLP, wherein each of said at least four ISVDs comprises three complementarity determining regions (CDR1 to CDR3, respectively), wherein the at least four ISVDs are optionally linked via one or more peptidic linkers, and wherein:
a first ISVD comprises:
   a CDR1 that is the amino acid sequence of SEQ ID NO: 7, a CDR2 that is the amino acid sequence of SEQ ID NO: 12 and a CDR3 that is the amino acid sequence of SEQ ID NO: 17,
a second ISVD comprises:
   a CDR1 that is the amino acid sequence of SEQ ID NO: 8, a CDR2 that is the amino acid sequence of SEQ ID NO: 13 and a CDR3 that is the amino acid sequence of SEQ ID NO: 18,
a third ISVD comprises:
   a CDR1 that is the amino acid sequence of SEQ ID NO: 9, a CDR2 that is the amino acid sequence of SEQ ID NO: 14 and a CDR3 that is the amino acid sequence of SEQ ID NO: 19, and
a fourth ISVD comprises:
   a CDR1 that is the amino acid sequence of SEQ ID NO: 11, a CDR2 that is the amino acid sequence of SEQ ID NO: 16 and a CDR3 that is the amino acid sequence of SEQ ID NO: 21.

In some embodiments, the compound that binds IL-13 and TSLP and which reduces the eosinophil count comprises or consists of the amino acid sequence of SEQ ID NO: 1.

### Increase of the FEV1

Increase of level the FEV1 indicates a better lung function and is thus beneficial for patients with pulmonary diseases, e.g., asthma. In some embodiments, the pulmonary disease is an inflammatory disease. In some embodiments, the pulmonary disease is asthma. In some embodiments, the asthma is high eosinophilic asthma. In some embodiments, the asthma is low eosinophilic asthma.

In the present invention, the increase in FEV1 (from baseline) is determined compared to a control, such a baseline or placebo. In some embodiments of the present invention, the FEV1 is increased by at least 0.05 L. In some embodiments of the present invention, the FEV1 is increased by at least 0.07 L. In some embodiments of the present invention, the FEV1 is increased by at least 0.1 L. In some embodiments of the present invention, the FEV1 is increased by at least 0.15 L. In some embodiments of the present invention, the FEV1 is increased by at least 0.2 L. In some embodiments of the present invention, the FEV1 is increased by at least 0.25 L. In some embodiments of the present invention, the FEV1 is increased by at least 0.3 L.

The present invention provides a compound that binds IL-13 and TSLP for use in treating a pulmonary disease, wherein the FEV1 is increased by at least 0.05 L compared to placebo. The present invention provides a compound that binds IL-13 and TSLP for use in treating a pulmonary disease, wherein the FEV1 is increased by at least 0.07 L compared to placebo. The present invention provides a compound that binds IL-13 and TSLP for use in treating a pulmonary disease, wherein the FEV1 is increased by at least 0.1 L compared to placebo.

In some embodiments, the increase in FEV1 occurs within 4 weeks after the administration of the compound. In some embodiments, the increase in FEV1 occurs within 2 weeks after the administration of the compound. In some embodiments, the increase in FEV1 occurs within 1 week after the administration of the compound.

In some embodiments, the compound that binds IL-13 and TSLP and which increases FEV1 is a polypeptide, such as an antibody or an antibody fragment. In some embodiments, the compound that binds IL-13 and TSLP and which increases FEV1 is a polypeptide, wherein the polypeptide comprises or consists of at least four ISVDs, wherein two ISVDs specifically bind IL-13 and two ISVDs specifically bind TSLP, wherein each of said at least four ISVDs comprises three complementarity determining regions (CDR1 to CDR3, respectively), wherein the at least four ISVDs are optionally linked via one or more peptidic linkers, and wherein:
a first ISVD comprises:
   a CDR1 that is the amino acid sequence of SEQ ID NO: 7, a CDR2 that is the amino acid sequence of SEQ ID NO: 12 and a CDR3 that is the amino acid sequence of SEQ ID NO: 17,
a second ISVD comprises:
   a CDR1 that is the amino acid sequence of SEQ ID NO: 8, a CDR2 that is the amino acid sequence of SEQ ID NO: 13 and a CDR3 that is the amino acid sequence of SEQ ID NO: 18,
a third ISVD comprises:
   a CDR1 that is the amino acid sequence of SEQ ID NO: 9, a CDR2 that is the amino acid sequence of SEQ ID NO: 14 and a CDR3 that is the amino acid sequence of SEQ ID NO: 19, and
a fourth ISVD comprises:
   a CDR1 that is the amino acid sequence of SEQ ID NO: 11, a CDR2 that is the amino acid sequence of SEQ ID NO: 16 and a CDR3 that is the amino acid sequence of SEQ ID NO: 21.

In some embodiments, the compound that binds IL-13 and TSLP and which increases FEV1 comprises or consists of the amino acid sequence of SEQ ID NO: 1.

### Reduction of airway inflammation

Reduction of airway inflammation indicates a better lung function and is thus beneficial for patients with pulmonary diseases, e.g., asthma. In some embodiments, airway inflammation is type 2 airway inflammation. In the present invention, the reduction of airway inflammation can be determined by analyzing clinical parameter, especially FeNO, eosinophil count and FEV1. The reduction or increase of those parameters is determined compared to a control which is either baseline or placebo (as explained above for the individual clinical parameters).

In some embodiments, airway inflammation is characterized by a reduction of FeNO by at least 18 ppb and a reduction of eosinophil count by at least 30%. In some embodiments, airway inflammation is characterized by a reduction of FeNO by at least 18 ppb, and an increase of the forced expiratory volume of the first second (FEV1) by at least 0.07 L. In some embodiments, airway inflammation is characterized by a reduction of eosinophil count by at least 30% and an increase of the forced expiratory volume of the first second (FEV1) by at least 0.07 L. In some embodiments, airway inflammation is characterized by a reduction of FeNO by at least 18 ppb, a reduction of eosinophil count by at least 30% and an increase of the forced expiratory volume of the first second (FEV1) by at least 0.07 L.

In some embodiments, airway inflammation is characterized by a reduction of FeNO by at least 20 ppb and a reduction of eosinophil count by at least 30%. In some embodiments, airway inflammation is characterized by a reduction of FeNO by at least 20 ppb, and an increase of the forced expiratory volume of the first second (FEV1) by at least 0.07 L. In some embodiments, airway inflammation is characterized by a reduction of FeNO by at least 20 ppb, a reduction of eosinophil count by at least 30% and an increase of the forced expiratory volume of the first second (FEV1) by at least 0.07 L.

In some embodiments, airway inflammation is characterized by a reduction of FeNO by at least 30 ppb and a reduction of eosinophil count by at least 30%. In some embodiments, airway inflammation is characterized by a reduction of FeNO by at least 30 ppb, and an increase of the forced expiratory volume of the first second (FEV1) by at least 0.07 L. In some embodiments, airway inflammation is characterized by a reduction of FeNO by at least 30 ppb, a reduction of eosinophil count by at least 30% and an increase of the forced expiratory volume of the first second (FEV1) by at least 0.07 L.

In some embodiments, airway inflammation is characterized by a reduction of FeNO by at least 40 ppb and a reduction of eosinophil count by at least 30%. In some embodiments, airway inflammation is characterized by a reduction of FeNO by at least 40 ppb, and an increase of the forced expiratory volume of the first second (FEV1) by at least 0.07 L. In some embodiments, airway inflammation is characterized by a reduction of FeNO by at least 40 ppb, a reduction of eosinophil count by at least 30% and an increase of the forced expiratory volume of the first second (FEV1) by at least 0.07 L.

In some embodiments, airway inflammation is characterized by a reduction of FeNO by at least 18 ppb, a reduction of eosinophil count by at least 30% and an increase of the forced expiratory volume of the first second (FEV1) by at least 0.07 L and a reduction of one or more of IL-5 level, CCL26 (eotaxin-3) level, TARC (CCL17) level and IgE level (serum). In some embodiments, airway inflammation is characterized by a reduction of FeNO by at least 18 ppb, a reduction of eosinophil count by at least 30% and an increase of the forced expiratory volume of the first second (FEV1) by at least 0.07 L and a reduction of IL-5 level, CCL26 (eotaxin-3) level, TARC (CCL17) level and IgE level.

In some embodiments, airway inflammation is characterized by a reduction of FeNO by at least 20 ppb, a reduction of eosinophil count by at least 30% and an increase of the forced expiratory volume of the first second (FEV1) by at least 0.07 L and a reduction of one or more of IL-5 level, CCL26 (eotaxin-3) level, TARC (CCL17) level and IgE level (serum). In some embodiments, airway inflammation is characterized by a reduction of FeNO by at least 20 ppb, a reduction of eosinophil count by at least 30% and an increase of the forced expiratory volume of the first second (FEV1) by at least 0.07 L and a reduction of IL-5 level, CCL26 (eotaxin-3) level, TARC (CCL17) level and IgE level.

In some embodiments, airway inflammation is characterized by a reduction of FeNO by at least 30 ppb, a reduction of eosinophil count by at least 30% and an increase of the forced expiratory volume of the first second (FEV1) by at least 0.07 L and a reduction of one or more of IL-5 level, CCL26 (eotaxin-3) level, TARC (CCL17) level and IgE level. In some embodiments, airway inflammation is characterized by a reduction of FeNO by at least 30 ppb, a reduction of eosinophil count by at least 30% and an increase of the forced expiratory volume of the first second (FEV1) by at least 0.07 L and a reduction of IL-5 level, CCL26 (eotaxin-3) level, TARC (CCL17) level and IgE level.

In some embodiments, airway inflammation is characterized by a reduction of FeNO by at least 40 ppb, a reduction of eosinophil count by at least 30% and an increase of the forced expiratory volume of the first second (FEV1) by at least 0.07 L and a reduction of one or more of IL-5 level, CCL26 (eotaxin-3) level, TARC (CCL17) level and IgE level (serum). In some embodiments, airway inflammation is characterized by a reduction of FeNO by at least 40 ppb, a reduction of eosinophil count by at least 30% and an increase of the forced expiratory volume of the first second (FEV1) by at least 0.07 L and a reduction of IL-5 level, CCL26 (eotaxin-3) level, TARC (CCL17) level and IgE level.

In some embodiments, the reduction of airway inflammation occurs within 4 weeks after the administration of the compound. In some embodiments, the reduction of airway inflammation occurs within 2 weeks after the administration of the compound. In some embodiments, the reduction of airway inflammation occurs within 1 week after the administration of the compound.

In some embodiments, the compound that binds IL-13 and TSLP and which reduces airway inflammation is a polypeptide, such as an antibody or an antibody fragment. In some embodiments, the compound that binds IL-13 and TSLP and which reduces airway inflammation is a polypeptide, wherein the polypeptide comprises or consists of at least four ISVDs, wherein two ISVDs specifically bind IL-13 and two ISVDs specifically bind TSLP, wherein each of said at least four ISVDs comprises three complementarity determining regions (CDR1 to CDR3, respectively), wherein the at least four ISVDs are optionally linked via one or more peptidic linkers, and wherein:
a first ISVD comprises:
   a CDR1 that is the amino acid sequence of SEQ ID NO: 7, a CDR2 that is the amino acid sequence of SEQ ID NO: 12 and a CDR3 that is the amino acid sequence of SEQ ID NO: 17,
a second ISVD comprises:
   a CDR1 that is the amino acid sequence of SEQ ID NO: 8, a CDR2 that is the amino acid sequence of SEQ ID NO: 13 and a CDR3 that is the amino acid sequence of SEQ ID NO: 18,
a third ISVD comprises:
   a CDR1 that is the amino acid sequence of SEQ ID NO: 9, a CDR2 that is the amino acid sequence of SEQ ID NO: 14 and a CDR3 that is the amino acid sequence of SEQ ID NO: 19, and
a fourth ISVD comprises:
   a CDR1 that is the amino acid sequence of SEQ ID NO: 11, a CDR2 that is the amino acid sequence of SEQ ID NO: 16 and a CDR3 that is the amino acid sequence of SEQ ID NO: 21.

In some embodiments, the compound that binds IL-13 and TSLP and which reduces airway inflammation comprises or consists of the amino acid sequence of SEQ ID NO: 1.

### Managing airway inflammation

The present invention can be used to manage airway inflammation. In some embodiments, managing airway inflammation comprises decreasing the FeNO level to a value of less than 40 ppb. In some embodiments, managing airway inflammation comprises decreasing the FeNO level to a value of less than 35. In some embodiments, managing airway inflammation comprises decreasing the FeNO level to a value of less than 30. In some embodiments, managing airway inflammation comprises decreasing the FeNO level to a value of less than 25. In some embodiments, managing airway inflammation comprises decreasing the FeNO level to a value of less than 20.

In some embodiments, managing airway inflammation comprises decreasing the FeNO level to a value of less than 40 ppb and to an eosinophil count in blood of less than 0.2 ^{∗}10⁹ cells/L. In some embodiments, managing airway inflammation comprises decreasing the FeNO level to a value of less than 35 ppb and to an eosinophil count in blood of less than 0.2 ^{∗}10⁹ cells/L. In some embodiments, managing airway inflammation comprises decreasing the FeNO level to a value of less than 30 ppb and to an eosinophil count in blood of less than 0.2 ^{∗}10⁹ cells/L. In some embodiments, managing airway inflammation comprises decreasing the FeNO level to a value of less than 25 ppb and to an eosinophil count in blood of less than 0.2 ^{∗}10⁹ cells/L. In some embodiments, managing airway inflammation comprises decreasing the FeNO level to a value of less than 20 ppb and to an eosinophil count in blood of less than 0.2 ^{∗}10⁹ cells/L.

In some embodiments, managing air way inflammation further comprises decreasing one or more of IL-5 level, CCL26 (eotaxin-3) level, TARC (CCL17) level and IgE level. In some embodiments, managing air way inflammation further comprises decreasing IL-5 level, CCL26 (eotaxin-3) level, TARC (CCL17) level and IgE level.

The present invention can be used to manage type 2 airway inflammation. In some embodiments, managing type 2 airway inflammation comprises decreasing the FeNO level to a value of less than 40 ppb. In some embodiments, managing type 2 airway inflammation comprises decreasing the FeNO level to a value of less than 35. In some embodiments, managing type 2 airway inflammation comprises decreasing the FeNO level to a value of less than 30. In some embodiments, managing type 2 airway inflammation comprises decreasing the FeNO level to a value of less than 25. In some embodiments, managing type 2 airway inflammation comprises decreasing the airway inflammation to a value of less than 20.

In some embodiments, managing type 2 airway inflammation comprises decreasing the FeNO level to a value of less than 40 ppb and to an eosinophil count in blood of less than 0.2 ^{∗}10⁹ cells/L. In some embodiments, managing type 2 airway inflammation comprises decreasing the FeNO level to a value of less than 35 ppb and to an eosinophil count in blood of less than 0.2 ^{∗}10⁹ cells/L. In some embodiments, managing type 2 airway inflammation comprises decreasing the FeNO level to a value of less than 30 ppb and to an eosinophil count in blood of less than 0.2 ^{∗}10⁹ cells/L. In some embodiments, managing type 2 airway inflammation comprises decreasing the FeNO level to a value of less than 25 ppb and to an eosinophil count in blood of less than 0.2 ^{∗}10⁹ cells/L. In some embodiments, managing type 2 airway inflammation comprises decreasing the FeNO level to a value of less than 20 ppb and to an eosinophil count in blood of less than 0.2 ^{∗}10⁹ cells/L.

### 5.3 Compounds for use according to the present invention

The compound that binds TSLP and IL-13 for use in the present invention can be a polypeptide. Suitable polypeptides have been described in the patent application WO2021116182, which is herewith incorporated in its entirety.

In some embodiments, the polypeptide is an antibody or an antibody fragment. Exemplary polypeptides for use in the present invention are polypeptides which comprise immunoglobulin single variable domains (ISVDs). ISVDs which bind TSLP and IL-13 can be found in Tables A-1 to A-6 of WO2021116182.

In some embodiments, the polypeptide comprises or consists of at least four ISVDs, wherein two ISVDs specifically bind IL-13 and two ISVDs specifically bind TSLP, wherein each of said at least four ISVDs comprises three complementarity determining regions (CDR1 to CDR3, respectively), wherein the at least four ISVDs are optionally linked via one or more peptidic linkers, and wherein:
a first ISVD comprises:
   a CDR1 that is the amino acid sequence of SEQ ID NO: 7, a CDR2 that is the amino acid sequence of SEQ ID NO: 12 and a CDR3 that is the amino acid sequence of SEQ ID NO: 17,
a second ISVD comprises:
   a CDR1 that is the amino acid sequence of SEQ ID NO: 8, a CDR2 that is the amino acid sequence of SEQ ID NO: 13 and a CDR3 that is the amino acid sequence of SEQ ID NO: 18,
a third ISVD comprises:
   a CDR1 that is the amino acid sequence of SEQ ID NO: 9, a CDR2 that is the amino acid sequence of SEQ ID NO: 14 and a CDR3 that is the amino acid sequence of SEQ ID NO: 19, and
a fourth ISVD comprises:
   a CDR1 that is the amino acid sequence of SEQ ID NO: 11, a CDR2 that is the amino acid sequence of SEQ ID NO: 16 and a CDR3 that is the amino acid sequence of SEQ ID NO: 21.

In some embodiments, the compound comprises:
a first ISVD comprising the amino acid sequence of SEQ ID NO: 2,
a second ISVD comprising the amino acid sequence of SEQ ID NO: 3,
a third ISVD comprising the amino acid sequence of SEQ ID NO: 4, and
a fourth ISVD comprising the amino acid sequence of SEQ ID NO: 6.

In some embodiment, the order of the ISVDs above indicates their relative position to each other considered from the N-terminus to the C-terminus of said polypeptide.

In some embodiments, the compound comprises a further ISVD that binds to human serum albumin, wherein the further ISVD comprises: a CDR1 that is the amino acid sequence of SEQ ID NO: 10; a CDR2 that is the amino acid sequence of SEQ ID NO: 15; and a CDR3 that is the amino acid sequence of SEQ ID NO: 20. In some embodiments, this further ISVD is positioned between the third and the forth ISVD above.

In some embodiments, the compound comprises:
a first ISVD comprising the amino acid sequence of SEQ ID NO: 2,
a second ISVD comprising the amino acid sequence of SEQ ID NO: 3,
a third ISVD comprising the amino acid sequence of SEQ ID NO: 4,
a fourth ISVD comprising the amino acid sequence of SEQ ID NO: 6,
and a further ISVD that binds to human serum albumin and comprises the amino acid sequence of SEQ ID NO: 5.

An exemplary polypeptide that binds TSLP and IL-13 is SAR443765 (SEQ ID NO: 1). In some embodiments, the polypeptide that binds TSLP and IL-13 is SAR443765 (SEQ ID NO: 1).

### 5.3.1 Immunoglobulin single variable domains

As stated above, in some embodiments the polypeptide for use in the present invention is a polypeptide which comprise immunoglobulin single variable domains (ISVDs).

The term "immunoglobulin single variable domain" (ISVD), interchangeably used with "single variable domain", defines immunoglobulin molecules wherein the antigen binding site is present on, and formed by, a single immunoglobulin domain. This sets ISVDs apart from "conventional" immunoglobulins (e.g., monoclonal antibodies) or their fragments (such as Fab, Fab', F(ab')₂, scFv, di-scFv), wherein two immunoglobulin domains, in particular two variable domains, interact to form an antigen binding site. Typically, in conventional immunoglobulins, a heavy chain variable domain (V_{H}) and a light chain variable domain (V_{L}) interact to form an antigen binding site. In this case, the complementarity determining regions (CDRs) of both V_{H} and V_{L} will contribute to the antigen binding site, i.e. a total of 6 CDRs will be involved in antigen binding site formation.

In view of the above definition, the antigen-binding domain of a conventional 4-chain antibody (such as an IgG, IgM, IgA, IgD or IgE molecule; known in the art) or of a Fab fragment, a F(ab')₂ fragment, an Fv fragment such as a disulfide linked Fv or a scFv fragment, or a diabody (all known in the art) derived from such conventional 4-chain antibody, would normally not be regarded as an ISVD, as, in these cases, binding to the respective epitope of an antigen would normally not occur by one (single) immunoglobulin domain but by a pair of (associating) immunoglobulin domains such as light and heavy chain variable domains, i.e., by a V_{H}-V_{L} pair of immunoglobulin domains, which jointly bind to an epitope of the respective antigen.

In contrast, ISVDs are capable of specifically binding to an epitope of the antigen without pairing with an additional immunoglobulin variable domain. The binding site of an ISVD is formed by a single V_{H}, a single V_{HH} or single V_{L} domain.

As such, the single variable domain may be a light chain variable domain sequence (e.g., a V_{L}-sequence) or a suitable fragment thereof; or a heavy chain variable domain sequence (e.g., a V_{H}-sequence or V_{HH} sequence) or a suitable fragment thereof; as long as it is capable of forming a single antigen binding unit (i.e., a functional antigen binding unit that essentially consists of the single variable domain, such that the single antigen binding domain does not need to interact with another variable domain to form a functional antigen binding unit).

An ISVD can for example be a heavy chain ISVD, such as a V_{H}, V_{HH}, including a camelized V_{H} or humanized V_{HH}. In one embodiment, it is a V_{HH}, including a camelized V_{H} or humanized V_{HH}. Heavy chain ISVDs can be derived from a conventional four-chain antibody or from a heavy chain antibody.

For example, the ISVD may be a single domain antibody (or an amino acid sequence that is suitable for use as a single domain antibody), a "dAb" or dAb (or an amino acid sequence that is suitable for use as a dAb) or a Nanobody^{®} (as defined herein, and including but not limited to a V_{HH}); other single variable domains, or any suitable fragment of any one thereof.

In particular, the ISVD may be a Nanobody^{®} (such as a V_{HH}, including a humanized V_{HH} or camelized V_{H}) or a suitable fragment thereof. Nanobody^{®}, Nanobodies^{®} and Nanoclone^{®} are registered trademarks of Sanofi or its affiliates.

"V_{HH} domains", also known as V_{HHS}, V_{HH} antibody fragments, and V_{HH} antibodies, have originally been described as the antigen binding immunoglobulin variable domain of "heavy chain antibodies" (i.e., of "antibodies devoid of light chains"; Hamers-Casterman et al. Nature 363:446-448, 1993). The term "V_{HH} domain" has been chosen in orderto distinguish these variable domains from the heavy chain variable domains that are present in conventional 4-chain antibodies (which are referred to herein as "V_{H} domains") and from the light chain variable domains that are present in conventional 4-chain antibodies (which are referred to herein as "V_{L} domains"). For a further description of V_{HH}'s, reference is made to the review article by Muyldermans (Reviews in Molecular Biotechnology 74: 277-302, 2001).

Typically, the generation of immunoglobulins involves the immunization of experimental animals, fusion of immunoglobulin producing cells to create hybridomas and screening for the desired specificities. Alternatively, immunoglobulins can be generated by screening of naïve or synthetic libraries e.g. by phage display.

The generation of immunoglobulin sequences, such as Nanobodies^{®}, has been described extensively in various publications, among which WO 94/04678, Hamers-Casterman et al. 1993 and Muyldermans, 2001 can be exemplified. In these methods, camelids are immunized with the target antigen in order to induce an immune response against said target antigen. The repertoire of Nanobodies obtained from said immunization is further screened for Nanobodies that bind the target antigen.

In these instances, the generation of antibodies requires purified antigen for immunization and/or screening. Antigens can be purified from natural sources, or in the course of recombinant production.

Immunization and/or screening for immunoglobulin sequences can be performed using peptide fragments of such antigens.

The present invention may use immunoglobulin sequences of different origin, comprising mouse, rat, rabbit, donkey, human and camelid immunoglobulin sequences. The present invention may also use fully human, humanized or chimeric sequences. For example, the present invention uses camelid immunoglobulin sequences and humanized camelid immunoglobulin sequences, or camelized domain antibodies, e.g. camelized dAb as described by Ward et al (see for example WO 94/04678 and Davies and Riechmann (1994 and 1996)). Moreover, the present invention also uses fused immunoglobulin sequences, e.g. forming a multivalent and/or multispecific construct (for multivalent and multispecific polypeptides containing one or more V_{HH} domains and their preparation, reference is also made to Conrath et al., J. Biol. Chem., Vol. 276, 10. 7346-7350, 2001, as well as to for example WO 96/34103 and WO 99/23221), and immunoglobulin sequences comprising tags or other functional moieties, e.g. toxins, labels, radiochemicals, etc., which are derivable from the immunoglobulin sequences of compound used in the present invention.

### 5.3.2 Antibodies

As stated above, in some embodiments the polypeptide for use in the present invention is an antibody. Antibodies which bind TSLP and IL-13 can be found in Venkataramani et al. (Biochem Biophys Res Commun. 2018 Sep 26;504(1):19-24.

### 5.3.3 Binding/Blocking

The compounds of the present invention bind to TSLP and IL-13. In some embodiments, the compound of the present invention can block its target molecules. For example, the compound can block the interaction between IL-13 and IL-13Rα1 (Interleukin 13 receptor, alpha 1) and/or the interaction between IL-13/IL-13Rα1 complex and IL-4Rα (alpha interleukin-4 receptor), and/or can block the interaction between TSLP and TSLPR (TSLP receptor) and/or TSLP/TSLPR complex and IL-7Rα (Interleukin-7 receptor subunit alpha). In some embodiments, the compound of the present invention can block the interaction between IL-13 and IL-13Rα1 (Interleukin 13 receptor, alpha 1) and/or the interaction between IL-13/IL-13Rα1 complex and IL-4Rα (alpha interleukin-4 receptor), and can block the interaction between TSLP and TSLPR (TSLP receptor) and/or TSLP/TSLPR complex and IL-7Rα (Interleukin-7 receptor subunit alpha).

In some embodiments, the compound for use in the present invention can bind human IL-13 (Uniprot accession P35225) and human TSLP (Uniprot accession Q969D9). In some embodiments, the compound for use in the present invention can bind IL-13 and TSLP from other mammals, for example IL-13 and TSLP from mice, rats, rabbits, cats, dogs, goats, sheep, horses, pigs, non-human primates, such as cynomolgus monkeys (also referred to herein as *"cyno"*), or camelids, such as llama or alpaca.

In relation to the compounds of the present invention, binding to IL-13 and TSLP means specific binding to IL-13 and TSLP. The binding of a compound to its target can be determined based on affinity. The affinity denotes the strength or stability of a molecular interaction. The affinity is commonly given as by the KD, or dissociation constant, which has units of mol/liter (or M). The affinity can also be expressed as an association constant, KA, which equals 1/KD and has units of (mol/liter)⁻¹ (or M⁻¹).

The affinity is a measure for the binding strength between a moiety and a binding site on the target molecule: the lower the value of the KD, the stronger the binding strength between a target molecule and a targeting moiety.

Typically, binding units used in the present invention will bind to their targets with a dissociation constant (KD) of 10⁻⁵ to 10⁻¹² moles/liter or less, or 10⁻⁷ to 10⁻¹² moles/liter or less, or 10⁻⁸ to 10⁻¹² moles/liter (i.e. with an association constant (KA) of 10⁵ to 10¹² liter/ moles or more, or 10⁷ to 10¹² liter/moles or more, or 10⁸ to 10¹² liter/moles).

Any KD value greater than 10⁻⁴ mol/liter (or any KA value lower than 10⁴ liters/mol) is generally considered to indicate non-specific interaction.

The KD for biological interactions, such as the binding of immunoglobulin sequences to an antigen, which are considered specific are typically in the range of 10⁻⁵ moles/liter (10000 nM or 10µM) to 10⁻¹² moles/liter (0.001 nM or 1 pM) or less.

Accordingly, specific/selective binding may mean that - using the same measurement method, e.g. SPR - a compound binds to IL13 and/or TSLP with a KD value of 10⁻⁵ to 10⁻¹² moles/liter or less and binds to related cytokines with a KD value greater than 10⁻⁴ moles/liter. Examples of IL13 related targets are human IL4. Examples of related cytokines for TSLP are human IL7. Thus, in some embodiments, the compound used in the present invention binds to IL13 with a KD value of 10⁻⁵ to 10⁻¹² moles/liter or less and binds to IL4 of the same species with a KD value greater than 10⁻⁴ moles/liter, and binds to TSLP with a KD value of 10⁻⁵ to 10⁻¹² moles/liter or less and binds to human IL7 of the same species with a KD value greater than 10⁻⁴ moles/liter.

In some embodiments, the polypeptides used in the present invention have at least half the binding affinity, or at least the same binding affinity, to human IL13 and to human TSLP as compared to a polypeptide consisting of the amino acid of SEQ ID NO: 1, wherein the binding affinity is measured using the same method, such as SPR.

Specific binding to a certain target from a certain species does not exclude that the binding unit can also specifically bind to the analogous target from a different species. For example, specific binding to human IL13 does not exclude that the binding unit (or a polypeptide comprising the same) can also specifically bind to IL13 from cynomolgus monkeys. Likewise, for example, specific binding to human TSLP does not exclude that the binding unit (or a polypeptide comprising the same) can also specifically bind to TSLP from cynomolgus monkeys ("cyno").

Specific binding of a binding unit to its designated target can be determined in any suitable manner known per se, including, for example, Scatchard analysis and/or competitive binding assays, such as radioimmunoassays (RIA), enzyme immunoassays (EIA) and sandwich competition assays, and the different variants thereof known per se in the art; as well as the other techniques mentioned herein.

The dissociation constant may be the actual or apparent dissociation constant, as will be clear to the skilled person. Methods for determining the dissociation constant will be clear to the skilled person, and for example include the techniques mentioned below. In this respect, it will also be clear that it may not be possible to measure dissociation constants of more than 10⁻⁴ moles/liter or 10⁻³ moles/liter (e.g. of 10⁻² moles/liter). Optionally, as will also be clear to the skilled person, the (actual or apparent) dissociation constant may be calculated on the basis of the (actual or apparent) association constant (KA), by means of the relationship [KD = 1/KA].

The affinity of a molecular interaction between two molecules can be measured via different techniques known *per se,* such as the well-known surface plasmon resonance (SPR) biosensor technique (see for example Ober et al. 2001, Intern. Immunology 13: 1551-1559). The term "surface plasmon resonance", as used herein, refers to an optical phenomenon that allows for the analysis of real-time biospecific interactions by detection of alterations in protein concentrations within a biosensor matrix, where one molecule is immobilized on the biosensor chip and the other molecule is passed over the immobilized molecule under flow conditions yielding kₒₙ, k_{off} measurements and hence K_{D} (or K_{A}) values. This can for example be performed using the well-known BIAcore^{®} system (BIAcore International AB, a GE Healthcare company, Uppsala, Sweden and Piscataway, NJ). For further descriptions, see Jonsson et al. (1993, Ann. Biol. Clin. 51: 19-26), Jonsson et al. (1991 Biotechniques 11: 620-627), Johnsson et al. (1995, J. Mol. Recognit. 8: 125-131), and Johnnson et al. (1991, Anal. Biochem. 198: 268-277).

Another well-known biosensor technique to determine affinities of biomolecular interactions is bio-layer interferometry (BLI) (see for example Abdiche et al. 2008, Anal. Biochem. 377: 209-217). The term "bio-layer Interferometry" or "BLI", as used herein, refers to a label-free optical technique that analyzes the interference pattern of light reflected from two surfaces: an internal reference layer (reference beam) and a layer of immobilized protein on the biosensor tip (signal beam). A change in the number of molecules bound to the tip of the biosensor causes a shift in the interference pattern, reported as a wavelength shift (nm), the magnitude of which is a direct measure of the number of molecules bound to the biosensor tip surface. Since the interactions can be measured in real-time, association and dissociation rates and affinities can be determined. BLI can for example be performed using the well-known Octet^{®} Systems (ForteBio, a division of Pall Life Sciences, Menlo Park, USA).

Alternatively, affinities can be measured in Kinetic Exclusion Assay (KinExA) (see for example Drake et al. 2004, Anal. Biochem., 328: 35-43), using the KinExA^{®} platform (Sapidyne Instruments Inc, Boise, USA). The term "KinExA", as used herein, refers to a solution-based method to measure true equilibrium binding affinity and kinetics of unmodified molecules. Equilibrated solutions of an antibody/antigen complex are passed over a column with beads precoated with antigen (or antibody), allowing the free antibody (or antigen) to bind to the coated molecule. Detection of the antibody (or antigen) thus captured is accomplished with a fluorescently labeled protein binding the antibody (or antigen).

The GYROLAB^{®} immunoassay system provides a platform for automated bioanalysis and rapid sample turnaround (Fraley et al. 2013, Bioanalysis 5: 1765-74).

### 5.3.4 Nucleic acid molecules

In some embodiments, the present invention relates to a nucleic acid for use in treating a pulmonary disease, wherein the nucleic acid encodes a compound that binds IL-13 and TSLP, wherein the treatment reduces the level of fractional exhaled nitric oxide (FeNO) by at least 18 ppb compared to placebo. In some embodiments, the pulmonary disease is an inflammatory disease. In some embodiments, the pulmonary disease is asthma. In some embodiments, the asthma is high eosinophilic asthma. In some embodiments, the asthma is low eosinophilic asthma.

A "nucleic acid molecule" (used interchangeably with "nucleic acid") is a chain of nucleotide monomers linked to each other via a phosphate backbone to form a nucleotide sequence. A nucleic acid may be used to transform/transfect a host cell or host organism, e.g. for expression and/or production of a polypeptide. Suitable hosts or host cells for production purposes will be clear to the skilled person, and may for example be any suitable fungal, prokaryotic or eukaryotic cell or cell line or any suitable fungal, prokaryotic or eukaryotic organism. A host or host cell comprising a nucleic acid encoding the polypeptide of the present invention is also encompassed by the present invention.

A nucleic acid may be for example DNA, RNA, or a hybrid thereof, and may also comprise (e.g., chemically) modified nucleotides, like PNA. It can be single- or double-stranded. In one embodiment, it is in the form of double-stranded DNA. For example, the nucleotide sequences of the present invention may be genomic DNA, cDNA.

The nucleic acids of the present invention can be prepared or obtained in a manner known per se, and/or can be isolated from a suitable natural source. Nucleotide sequences encoding naturally occurring (poly)peptides can for example be subjected to site-directed mutagenesis, so as to provide a nucleic acid molecule encoding polypeptide with sequence variation. Also, as will be clear to the skilled person, to prepare a nucleic acid, also several nucleotide sequences, such as at least one nucleotide sequence encoding a targeting moiety and for example nucleic acids encoding one or more linkers can be linked together in a suitable manner.

Techniques for generating nucleic acids will be clear to the skilled person and may for instance include, but are not limited to, automated DNA synthesis; site-directed mutagenesis; combining two or more naturally occurring and/or synthetic sequences (or two or more parts thereof), introduction of mutations that lead to the expression of a truncated expression product; introduction of one or more restriction sites (e.g. to create cassettes and/or regions that may easily be digested and/or ligated using suitable restriction enzymes), and/or the introduction of mutations by means of a PCR reaction using one or more "mismatched" primers.

### 5.3.5 Compositions

The present invention also provides a pharmaceutical composition for use in the present invention, wherein the pharmaceutical composition comprises at least one compound that binds to TSLP and IL-13, and/or at least one nucleic acid molecule encoding a compound that binds to TSLP and IL-13. The composition may further comprise at least one pharmaceutically acceptable carrier, diluent or excipient and/or adjuvant, and optionally comprise one or more further pharmaceutically active polypeptides and/or compounds.

In some embodiments, the invention provides a pharmaceutical composition comprising a compound that binds IL-13 and TSLP for use in treating a pulmonary disease, wherein the composition further comprises at least one pharmaceutically acceptable carrier, diluent or excipient and/or adjuvant, and optionally comprises one or more further pharmaceutically active polypeptides and/or compounds, wherein the treatment reduces the level of fractional exhaled nitric oxide (FeNO) by at least 18 ppb compared to placebo. In some embodiments, the pulmonary disease is an inflammatory disease. In some embodiments, the pulmonary disease is asthma. In some embodiments, the asthma is high eosinophilic asthma. In some embodiments, the asthma is low eosinophilic asthma.

In some embodiments, the invention provides a pharmaceutical composition comprising a compound that binds IL-13 and TSLP for use in treating a pulmonary disease, wherein the composition further comprises at least one pharmaceutically acceptable carrier, diluent or excipient and/or adjuvant, and optionally comprises one or more further pharmaceutically active polypeptides and/or compounds, wherein the treatment reduces the level of fractional exhaled nitric oxide (FeNO) by at least 20 ppb compared to placebo. In some embodiments, the pulmonary disease is an inflammatory disease. In some embodiments, the pulmonary disease is asthma. In some embodiments, the asthma is high eosinophilic asthma. In some embodiments, the asthma is low eosinophilic asthma.

In some embodiments, the invention provides a pharmaceutical composition comprising a compound that binds IL-13 and TSLP for use in treating a pulmonary disease, wherein the composition further comprises at least one pharmaceutically acceptable carrier, diluent or excipient and/or adjuvant, and optionally comprises one or more further pharmaceutically active polypeptides and/or compounds, wherein the treatment reduces the level of fractional exhaled nitric oxide (FeNO) by at least 30 ppb compared to placebo. In some embodiments, the pulmonary disease is an inflammatory disease. In some embodiments, the pulmonary disease is asthma. In some embodiments, the asthma is high eosinophilic asthma. In some embodiments, the asthma is low eosinophilic asthma.

In some embodiments, the invention provides a pharmaceutical composition comprising a compound that binds IL-13 and TSLP for use in treating a pulmonary disease, wherein the composition further comprises at least one pharmaceutically acceptable carrier, diluent or excipient and/or adjuvant, and optionally comprises one or more further pharmaceutically active polypeptides and/or compounds, wherein the treatment reduces the level of fractional exhaled nitric oxide (FeNO) by at least 40 ppb compared to placebo. In some embodiments, the pulmonary disease is an inflammatory disease. In some embodiments, the pulmonary disease is asthma. In some embodiments, the asthma is high eosinophilic asthma. In some embodiments, the asthma is low eosinophilic asthma.

### 5.4 Embodiments of the invention

In the following exemplary embodiments of the present invention are given for illustrative purposes. These embodiments relate to a compound that binds IL-13 and TSLP for use in the treatment of a pulmonary disease, wherein said treatment reduces the level of fractional exhaled nitric oxide (FeNO) by at least 18 ppb compared to a control.

### FeNO level + time points

In some embodiments, the treatment reduces the level of fractional exhaled nitric oxide (FeNO) by at least 18 ppb compared to placebo within 4 weeks after the administration of the compound.

In some embodiments, the treatment reduces the level of fractional exhaled nitric oxide (FeNO) by at least 18 ppb compared to placebo within 2 weeks after the administration of the compound.

In some embodiments, the treatment reduces the level of fractional exhaled nitric oxide (FeNO) by at least 18 ppb compared to placebo within 1 week after the administration of the compound.

In some embodiments, the treatment reduces the level of fractional exhaled nitric oxide (FeNO) by at least 20 ppb compared to placebo within 4 weeks after the administration of the compound.

In some embodiments, the treatment reduces the level of fractional exhaled nitric oxide (FeNO) by at least 20 ppb compared to placebo within 2 weeks after the administration of the compound.

In some embodiments, the treatment reduces the level of fractional exhaled nitric oxide (FeNO) by at least 20 ppb compared to placebo within 1 week after the administration of the compound.

In some embodiments, the treatment reduces the level of fractional exhaled nitric oxide (FeNO) by at least 25 ppb compared to placebo within 4 weeks after the administration of the compound.

In some embodiments, the treatment reduces the level of fractional exhaled nitric oxide (FeNO) by at least 25 ppb compared to placebo within 2 weeks after the administration of the compound.

In some embodiments, the treatment reduces the level of fractional exhaled nitric oxide (FeNO) by at least 25 ppb compared to placebo within 1 week after the administration of the compound.

In some embodiments, the treatment reduces the level of fractional exhaled nitric oxide (FeNO) by at least 30 ppb compared to placebo within 4 weeks after the administration of the compound.

In some embodiments, the treatment reduces the level of fractional exhaled nitric oxide (FeNO) by at least 30 ppb compared to placebo within 2 weeks after the administration of the compound.

In some embodiments, the treatment reduces the level of fractional exhaled nitric oxide (FeNO) by at least 30 ppb compared to placebo within 1 week after the administration of the compound.

In some embodiments, the treatment reduces the level of fractional exhaled nitric oxide (FeNO) by at least 35 ppb compared to placebo within 4 weeks after the administration of the compound.

In some embodiments, the treatment reduces the level of fractional exhaled nitric oxide (FeNO) by at least 35 ppb compared to placebo within 2 weeks after the administration of the compound.

In some embodiments, the treatment reduces the level of fractional exhaled nitric oxide (FeNO) by at least 35 ppb compared to placebo within 1 week after the administration of the compound.

In some embodiments, the treatment reduces the level of fractional exhaled nitric oxide (FeNO) by at least 40 ppb compared to placebo within 4 weeks after the administration of the compound.

In some embodiments, the treatment reduces the level of fractional exhaled nitric oxide (FeNO) by at least 40 ppb compared to placebo within 2 weeks after the administration of the compound.

In some embodiments, the treatment reduces the level of fractional exhaled nitric oxide (FeNO) by at least 40 ppb compared to placebo within 1 week after the administration of the compound.

### FeNO baseline levels

In some embodiments, the treatment is applied to patients having a baseline (before the administration of the compound) FeNO level of at least 25.

In some embodiments, the treatment is applied to patients having a baseline (before the administration of the compound) FeNO level of at least 30.

In some embodiments, the treatment is applied to patients having a baseline (before the administration of the compound) FeNO level of at least 40.

In some embodiments, the treatment is applied to patients having a baseline (before the administration of the compound) FeNO level of at least 50.

In some embodiments, the treatment is applied to patients having a baseline (before the administration of the compound) FeNO level of at least 60.

In some embodiments, the treatment is applied to patients having a baseline (before the administration of the compound) FeNO level of at least 70.

In some embodiments, the treatment is applied to patients for managing a baseline (before the administration of the compound) FeNO level of at least 25.

In some embodiments, the treatment is applied to patients for managing a baseline (before the administration of the compound) FeNO level of at least 30.

In some embodiments, the treatment is applied to patients for managing a baseline (before the administration of the compound) FeNO level of at least 40.

In some embodiments, the treatment is applied to patients for managing a baseline (before the administration of the compound) FeNO level of at least 50.

In some embodiments, the treatment is applied to patients for managing a baseline (before the administration of the compound) FeNO level of at least 60.

In some embodiments, the treatment is applied to patients for managing a baseline (before the administration of the compound) FeNO level of at least 70.

### Eosinophil count + time points

In some embodiments of the present invention, the eosinophil count is reduced by at least 30% compared to placebo within 4 weeks after the administration of the compound.

In some embodiments of the present invention, the eosinophil count is reduced by at least 30% compared to placebo within 2 weeks after the administration of the compound.

In some embodiments of the present invention, the eosinophil count is reduced by at least 30% compared to placebo within 1 week after the administration of the compound.

In some embodiments of the present invention, the eosinophil count is reduced by at least 30% compared to placebo within 3 days after the administration of the compound.

In some embodiments of the present invention, the eosinophil count is reduced by at least 30% compared to placebo within 1 day after the administration of the compound.

In some embodiments of the present invention, the eosinophil count is reduced by at least 35% compared to placebo within 4 weeks after the administration of the compound.

In some embodiments of the present invention, the eosinophil count is reduced by at least 35% compared to placebo within 2 weeks after the administration of the compound.

In some embodiments of the present invention, the eosinophil count is reduced by at least 35% compared to placebo within 1 week after the administration of the compound.

In some embodiments of the present invention, the eosinophil count is reduced by at least 35% compared to placebo within 3 days after the administration of the compound.

In some embodiments of the present invention, the eosinophil count is reduced by at least 35% compared to placebo within 1 day after the administration of the compound.

In some embodiments of the present invention, the eosinophil count is reduced by at least 40% compared to placebo within 4 weeks after the administration of the compound.

In some embodiments of the present invention, the eosinophil count is reduced by at least 40% compared to placebo within 2 weeks after the administration of the compound.

In some embodiments of the present invention, the eosinophil count is reduced by at least 40% compared to placebo within 1 week after the administration of the compound.

In some embodiments of the present invention, the eosinophil count is reduced by at least 40% compared to placebo within 3 days after the administration of the compound.

In some embodiments of the present invention, the eosinophil count is reduced by at least 40% compared to placebo within 1 day after the administration of the compound.

### FENO levels + eosinophil count + time points

In some embodiments, the treatment reduces the level of fractional exhaled nitric oxide (FeNO) by at least 18 ppb and reduces the eosinophil count by at least 30% compared to placebo within 4 weeks after the administration of the compound.

In some embodiments, the treatment reduces the level of fractional exhaled nitric oxide (FeNO) by at least 20 ppb and reduces the eosinophil count by at least 30% compared to placebo within 4 weeks after the administration of the compound.

In some embodiments, the treatment reduces the level of fractional exhaled nitric oxide (FeNO) by at least 30 ppb and reduces the eosinophil count by at least 30% compared to placebo within 4 weeks after the administration of the compound.

In some embodiments, the treatment reduces the level of fractional exhaled nitric oxide (FeNO) by at least 40 ppb and reduces the eosinophil count by at least 30% compared to placebo within 4 weeks after the administration of the compound.

In some embodiments, the treatment reduces the level of fractional exhaled nitric oxide (FeNO) by at least 18 ppb and reduces the eosinophil count by at least 30% compared to placebo within 2 weeks after the administration of the compound.

In some embodiments, the treatment reduces the level of fractional exhaled nitric oxide (FeNO) by at least 20 ppb and reduces the eosinophil count by at least 30% compared to placebo within 2 weeks after the administration of the compound.

In some embodiments, the treatment reduces the level of fractional exhaled nitric oxide (FeNO) by at least 30 ppb and reduces the eosinophil count by at least 30% compared to placebo within 2 weeks after the administration of the compound.

In some embodiments, the treatment reduces the level of fractional exhaled nitric oxide (FeNO) by at least 40 ppb and reduces the eosinophil count by at least 30% compared to placebo within 2 weeks after the administration of the compound.

In some embodiments, the treatment reduces the level of fractional exhaled nitric oxide (FeNO) by at least 18 ppb and reduces the eosinophil count by at least 30% compared to placebo within 1 week after the administration of the compound.

In some embodiments, the treatment reduces the level of fractional exhaled nitric oxide (FeNO) by at least 20 ppb and reduces the eosinophil count by at least 30% compared to placebo within 1 week after the administration of the compound.

In some embodiments, the treatment reduces the level of fractional exhaled nitric oxide (FeNO) by at least 30 ppb and reduces the eosinophil count by at least 30% compared to placebo within 1 week after the administration of the compound.

In some embodiments, the treatment reduces the level of fractional exhaled nitric oxide (FeNO) by at least 40 ppb and reduces the eosinophil count by at least 30% compared to placebo within 1 week after the administration of the compound.

### SEQ ID NO: 1 + asthma

In some embodiments, the present invention provides a compound that binds IL-13 and TSLP for use in treating asthma, wherein said compound is a polypeptide which comprises or consists of the amino acid sequence of SEQ ID NO: 1.

In some embodiments, the present invention provides a compound that binds IL-13 and TSLP for use in treating high eosinophilic asthma, wherein said compound is a polypeptide which comprises or consists of the amino acid sequence of SEQ ID NO: 1.

In some embodiments, the present invention provides a compound that binds IL-13 and TSLP for use in treating low eosinophilic asthma, wherein said compound is a polypeptide which comprises or consists of the amino acid sequence of SEQ ID NO: 1.

### SEQ ID NO: 1 + asthma + FeNO level

In some embodiments, the present invention provides a compound that binds IL-13 and TSLP for use in treating asthma, wherein the treatment reduces the level of fractional exhaled nitric oxide (FeNO) by at least 18 ppb compared to placebo, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces the level of fractional exhaled nitric oxide (FeNO) by at least 20 ppb compared to placebo, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces the level of fractional exhaled nitric oxide (FeNO) by at least 30 ppb compared to placebo, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces the level of fractional exhaled nitric oxide (FeNO) by at least 40 ppb compared to placebo, wherein said compound is a polypeptide which comprises or consists of the amino acid sequence of SEQ ID NO: 1.

### SEQ ID NO: 1 + asthma + FeNO level + time points

In some embodiments, the present invention provides a compound that binds IL-13 and TSLP for use in treating asthma, wherein the treatment reduces the level of fractional exhaled nitric oxide (FeNO) by at least 18 ppb compared to a control, wherein said reduction of the FenO level occurs within 4 weeks after the administration of the compound, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces the level of fractional exhaled nitric oxide (FeNO) by at least 20 ppb compared to compared to a control, wherein said reduction of the FenO level occurs within 4 weeks after the administration of the compound, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces the level of fractional exhaled nitric oxide (FeNO) by at least 30 ppb compared to a control, wherein said reduction of the FenO level occurs within 4 weeks after the administration of the compound, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces the level of fractional exhaled nitric oxide (FeNO) by at least 40 ppb compared to a control, wherein said reduction of the FenO level occurs within 4 weeks after the administration of the compound, wherein said compound is a polypeptide which comprises or consists of the amino acid sequence of SEQ ID NO: 1.

### SEQ ID NO: 1 + asthma + FeNO level + time points + placebo

In some embodiments, the present invention provides a compound that binds IL-13 and TSLP for use in treating asthma, wherein the treatment reduces the level of fractional exhaled nitric oxide (FeNO) by at least 18 ppb compared to placebo, wherein said reduction of the FenO level occurs within 4 weeks after the administration of the compound, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces the level of fractional exhaled nitric oxide (FeNO) by at least 20 ppb compared to placebo, wherein said reduction of the FenO level occurs within 4 weeks after the administration of the compound, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces the level of fractional exhaled nitric oxide (FeNO) by at least 30 ppb compared to placebo, wherein said reduction of the FenO level occurs within 4 weeks after the administration of the compound, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces the level of fractional exhaled nitric oxide (FeNO) by at least 40 ppb compared to placebo, wherein said reduction of the FenO level occurs within 4 weeks after the administration of the compound, wherein said compound is a polypeptide which comprises or consists of the amino acid sequence of SEQ ID NO: 1.

### SEQ ID NO: 1 + asthma + eosinophil count + time points

In some embodiments, the present invention provides a compound that binds IL-13 and TSLP for use in treating asthma, wherein the treatment reduces the eosinophil count in blood by at least 30% compared to a control, wherein said reduction of the eosinophil count occurs within 4 weeks after the administration of the compound, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces the eosinophil count in blood by at least 35% compared to a control, wherein said reduction of the eosinophil count occurs within 4 weeks after the administration of the compound, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces the eosinophil count in blood by at least 40% compared to a control, wherein said reduction of the eosinophil count occurs within 4 weeks after the administration of the compound, wherein said compound is a polypeptide which comprises or consists of the amino acid sequence of SEQ ID NO: 1.

### SEQ ID NO: 1 + asthma + eosinophil count + time points + placebo

In some embodiments, the present invention provides a compound that binds IL-13 and TSLP for use in treating asthma, wherein the treatment reduces the eosinophil count in blood by at least 30% compared to placebo, wherein said reduction of the eosinophil count occurs within 4 weeks after the administration of the compound, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces the eosinophil count in blood by at least 35% compared to placebo, wherein said reduction of the eosinophil count occurs within 4 weeks after the administration of the compound, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces the eosinophil count in blood by at least 40% compared to placebo, wherein said reduction of the eosinophil count occurs within 4 weeks after the administration of the compound, wherein said compound is a polypeptide which comprises or consists of the amino acid sequence of SEQ ID NO: 1.

### SEQ ID NO: 1 + asthma + airway inflammation + time points

In some embodiments, the present invention provides a compound that binds IL-13 and TSLP for use in treating asthma, wherein the treatment reduces airway inflammation wherein said reduction of airway inflammation is characterized by a reduction of FeNO by at least 18 ppb, a reduction of eosinophil count by at least 30% and/or an increase of the forced expiratory volume in one second (FEV1) by at least 0.07 L compared to a control, wherein said reduction of airway inflammation occurs within 4 weeks after the administration of the compound, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces airway inflammation wherein said reduction of airway inflammation is characterized by a reduction of FeNO by at least 20 ppb, a reduction of eosinophil count by at least 30% and/or an increase of the forced expiratory volume in one second (FEV1) by at least 0.07 L compared to a control, wherein said reduction of airway inflammation occurs within 4 weeks after the administration of the compound, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces airway inflammation wherein said reduction of airway inflammation is characterized by a reduction of FeNO by at least 30 ppb, a reduction of eosinophil count by at least 30% and/or an increase of the forced expiratory volume in one second (FEV1) by at least 0.07 L compared to a control, wherein said reduction of airway inflammation occurs within 4 weeks after the administration of the compound, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces airway inflammation wherein said reduction of airway inflammation is characterized by a reduction of FeNO by at least 40 ppb, a reduction of eosinophil count by at least 30% and/or an increase of the forced expiratory volume in one second (FEV1) by at least 0.07 L compared to a control, wherein said reduction of airway inflammation occurs within 4 weeks after the administration of the compound, wherein said compound is a polypeptide which comprises or consists of the amino acid sequence of SEQ ID NO: 1.

### SEQ ID NO: 1 + asthma + airway inflammation + time points + placebo

In some embodiments, the present invention provides a compound that binds IL-13 and TSLP for use in treating asthma, wherein the treatment reduces airway inflammation wherein said reduction of airway inflammation is characterized by a reduction of FeNO by at least 18 ppb, a reduction of eosinophil count by at least 30% and/or an increase of the forced expiratory volume in one second (FEV1) by at least 0.07 L compared to placebo, wherein said reduction of airway inflammation occurs within 4 weeks after the administration of the compound, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces airway inflammation wherein said reduction of airway inflammation is characterized by a reduction of FeNO by at least 20 ppb, a reduction of eosinophil count by at least 30% and/or an increase of the forced expiratory volume in one second (FEV1) by at least 0.07 L compared to placebo, wherein said reduction of airway inflammation occurs within 4 weeks after the administration of the compound, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces airway inflammation wherein said reduction of airway inflammation is characterized by a reduction of FeNO by at least 30 ppb, a reduction of eosinophil count by at least 30% and/or an increase of the forced expiratory volume in one second (FEV1) by at least 0.07 L compared to placebo, wherein said reduction of airway inflammation occurs within 4 weeks after the administration of the compound, wherein said compound is a polypeptide which comprises or consists of the amino acid sequence of SEQ ID NO: 1.

In some embodiments, the present invention provides a compound that binds IL-13 and TSLP for use in treating asthma, wherein the treatment reduces airway inflammation wherein said reduction of airway inflammation is characterized by a reduction of FeNO by at least 40 ppb, a reduction of eosinophil count by at least 30% and/or an increase of the forced expiratory volume in one second (FEV1) by at least 0.07 L compared to placebo, wherein said reduction of airway inflammation occurs within 4 weeks after the administration of the compound, wherein said compound is a polypeptide which comprises or consists of the amino acid sequence of SEQ ID NO: 1.

### Methods of treatment of a pulmonary disease

The present invention also provides methods for treatment of a pulmonary disease, comprising administering to a subject an effective amount of a compound that binds IL-13 and TSLP, wherein said treatment reduces the level of fractional exhaled nitric oxide (FeNO) compared to a control.

In some embodiments, the invention provides methods for treatment of a pulmonary disease, comprising administering to a subject an effective amount of a compound that binds IL-13 and TSLP, wherein said treatment reduces the level of fractional exhaled nitric oxide (FeNO) by at least 18 ppb compared to a control.

All embodiments and examples given above for compounds that binds IL-13 and TSLP for use in the treatment of a pulmonary disease, wherein said treatment reduces the level of fractional exhaled nitric oxide (FeNO) compared to a control, can be transferred to methods for treatment of a pulmonary disease, comprising administering to a subject an effective amount of a compound that binds IL-13 and TSLP, wherein said treatment reduces the level of fractional exhaled nitric oxide (FeNO) compared to a control. This applies in particular to reduction of FeNO level, time points, the nature of the compound, the type of pulmonary diseases, and the control. E.g., in some embodiments, the invention provides a method for treatment of a pulmonary disease, comprising administering to a subject an effective amount of a compound that binds IL-13 and TSLP, wherein said treatment reduces the level of fractional exhaled nitric oxide (FeNO) by at least 20 ppb, at least 30 ppb, or by at least 40 ppb compared to a control.

The present invention also provides methods for treatment of a pulmonary disease, comprising administering to a subject an effective amount of a compound that binds IL-13 and TSLP, wherein said treatment reduces the eosinophil count in blood compared to a control.

In some embodiments, the invention provides methods for treatment of a pulmonary disease, comprising administering to a subject an effective amount of a compound that binds IL-13 and TSLP, wherein said treatment reduces the eosinophil count in blood by at least 30% compared to a control.

All embodiments and examples given above for compounds that binds IL-13 and TSLP for use in the treatment of a pulmonary disease, wherein said treatment reduces the eosinophil count in blood compared to a control, can be transferred to methods for treatment of a pulmonary disease, comprising administering to a subject an effective amount of a compound that binds IL-13 and TSLP, wherein said treatment reduces the eosinophil count in blood compared to a control. This applies in particularto the reduction of eosinophil count, time points, the nature of the compound, the type of pulmonary diseases, and the control. E.g., in some embodiments, the invention provides a method for treatment of a pulmonary disease, comprising administering to a subject an effective amount of a compound that binds IL-13 and TSLP, wherein said treatment reduces the eosinophil count in blood by at least 35% or by at least 40% compared to a control.

The invention also provides methods for treatment of a pulmonary disease, comprising administering to a subject an effective amount of a compound that binds IL-13 and TSLP, wherein said treatment reduces airway inflammation. All embodiments and examples given above for compounds that binds IL-13 and TSLP for use in the treatment of a pulmonary disease, wherein said treatment reduces airway inflammation, can be transferred to methods for treatment of a pulmonary disease, comprising administering to a subject an effective amount of a compound that binds IL-13 and TSLP, wherein said treatment reduces airway inflammation. This applies in particular to the definition of reduction of airway inflammation, time points, the nature of the compound, the type of pulmonary diseases, and the control.

### Use of a compound that binds IL-13 and TSLP in the preparation of a pharmaceutical composition for the treatment of a pulmonary disease

The present invention also provides the use of a compound that binds IL-13 and TSLP in the preparation of a pharmaceutical composition for the treatment of a pulmonary disease, wherein said treatment reduces the level of fractional exhaled nitric oxide (FeNO) compared to a control.

In some embodiments, the invention provides the use of a compound that binds IL-13 and TSLP in the preparation of a pharmaceutical composition for the treatment of a pulmonary disease, wherein said treatment reduces the level of fractional exhaled nitric oxide (FeNO) by at least 18 ppb compared to a control.

All embodiments and examples given above for compounds that binds IL-13 and TSLP for use in the treatment of a pulmonary disease, wherein said treatment reduces the level of fractional exhaled nitric oxide (FeNO) compared to a control, can be transferred to the use of a compound that binds IL-13 and TSLP in the preparation of a pharmaceutical composition for the treatment of a pulmonary disease, wherein said treatment reduces the level of fractional exhaled nitric oxide (FeNO) compared to a control. This applies in particular to reduction of FeNO level, time points, the nature of the compound, the type of pulmonary diseases, and the control. E.g., in some embodiments, the invention provides the use of a compound that binds IL-13 and TSLP in the preparation of a pharmaceutical composition for the treatment of a pulmonary disease, wherein said treatment reduces the level of fractional exhaled nitric oxide (FeNO) by at least 20 ppb, at least 30 ppb, or by at least 40 ppb compared to a control.

The present invention also provides the use of a compound that binds IL-13 and TSLP in the preparation of a pharmaceutical composition for the treatment of a pulmonary disease, wherein said treatment reduces the eosinophil count in blood compared to a control.

In some embodiments, the invention provides the use of a compound that binds IL-13 and TSLP in the preparation of a pharmaceutical composition for the treatment of a pulmonary disease, wherein said treatment reduces the eosinophil count in blood by at least 30% compared to a control.

All embodiments and examples given above for compounds that binds IL-13 and TSLP for use in the treatment of a pulmonary disease, wherein said treatment reduces the eosinophil count in blood compared to a control, can be transferred to the use of a compound that binds IL-13 and TSLP in the preparation of a pharmaceutical composition for the treatment of a pulmonary disease, wherein said treatment reduces the eosinophil count in blood compared to a control. This applies in particular to the reduction of eosinophil count, time points, the nature of the compound, the type of pulmonary diseases, and the control. E.g., in some embodiments, the invention provides the use of a compound that binds IL-13 and TSLP in the preparation of a pharmaceutical composition for the treatment of a pulmonary disease, wherein said treatment reduces the eosinophil count in blood by at least 35% or by at least 40% compared to a control.

The invention also provides the use of a compound that binds IL-13 and TSLP in the preparation of a pharmaceutical composition for the treatment of a pulmonary disease, wherein said treatment reduces airway inflammation. All embodiments and examples given above for compounds that binds IL-13 and TSLP for use in the treatment of a pulmonary disease, wherein said treatment reduces airway inflammation, can be transferred to the use of a compound that binds IL-13 and TSLP in the preparation of a pharmaceutical composition for the treatment of a pulmonary disease, wherein said treatment reduces airway inflammation. This applies in particular to the definition of reduction of airway inflammation, time points, the nature of the compound, the type of pulmonary diseases, and the control.

### Use of a compound that binds IL-13 and TSLP for the treatment of a pulmonary disease

The present invention also provides the use of a compound that binds IL-13 and TSLP for the treatment of a pulmonary disease, wherein said treatment reduces the level of fractional exhaled nitric oxide (FeNO) compared to a control.

In some embodiments, the invention provides the use of a compound that binds IL-13 and TSLP for the treatment of a pulmonary disease, wherein said treatment reduces the level of fractional exhaled nitric oxide (FeNO) by at least 18 ppb compared to a control.

All embodiments and examples given above for compounds that binds IL-13 and TSLP for use in the treatment of a pulmonary disease, wherein said treatment reduces the level of fractional exhaled nitric oxide (FeNO) compared to a control, can be transferred to the use of a compound that binds IL-13 and TSLP for the treatment of a pulmonary disease, wherein said treatment reduces the level of fractional exhaled nitric oxide (FeNO) compared to a control. This applies in particular to reduction of FeNO level, time points, the nature of the compound, the type of pulmonary diseases, and the control. E.g., in some embodiments, the invention provides the use of a compound that binds IL-13 and TSLP for the treatment of a pulmonary disease, wherein said treatment reduces the level of fractional exhaled nitric oxide (FeNO) by at least 20 ppb, at least 30 ppb, or by at least 40 ppb compared to a control.

The present invention also provides the use of a compound that binds IL-13 and TSLP for the treatment of a pulmonary disease, wherein said treatment reduces the eosinophil count in blood compared to a control.

In some embodiments, the invention provides the use of a compound that binds IL-13 and TSLP for the treatment of a pulmonary disease, wherein said treatment reduces the eosinophil count in blood by at least 30% compared to a control.

All embodiments and examples given above for compounds that binds IL-13 and TSLP for use in the treatment of a pulmonary disease, wherein said treatment reduces the eosinophil count in blood compared to a control, can be transferred to the use of a compound that binds IL-13 and TSLP for the treatment of a pulmonary disease, wherein said treatment reduces the eosinophil count in blood compared to a control. This applies in particular to the reduction of eosinophil count, time points, the nature of the compound, the type of pulmonary diseases, and the control. E.g., in some embodiments, the invention provides the use of a compound that binds IL-13 and TSLP for the treatment of a pulmonary disease, wherein said treatment reduces the eosinophil count in blood by at least 35% or by at least 40% compared to a control.

The invention also provides the use of a compound that binds IL-13 and TSLP for the treatment of a pulmonary disease, wherein said treatment reduces airway inflammation. All embodiments and examples given above for compounds that binds IL-13 and TSLP for use in the treatment of a pulmonary disease, wherein said treatment reduces airway inflammation, can be transferred to the use of a compound that binds IL-13 and TSLP for the treatment of a pulmonary disease, wherein said treatment reduces airway inflammation. This applies in particular to the definition of reduction of airway inflammation, time points, the nature of the compound, the type of pulmonary diseases, and the control.

### Method of reducing the FeNO level

The present invention also provides compounds for use in reducing the FeNO level in a subject and methods for reducing the FeNO level in a subject.

In some embodiments, the invention provides a compound that binds IL-13 and TSLP for use in reducing the level of fractional exhaled nitric oxide (FeNO) in a subject, wherein said compound reduces the level of FeNO by at least 18 ppb compared to a control. The embodiments and examples given above for compounds that binds IL-13 and TSLP for use in the treatment of a pulmonary disease, wherein said treatment reduces the level of fractional exhaled nitric oxide (FeNO) compared to a control, can be transferred to compounds that binds IL-13 and TSLP for use in reducing the level of fractional exhaled nitric oxide (FeNO) in a subject. This applies in particular to the reduction of FeNO level, time points, the nature of the compound, and the control. E.g., in some embodiments, the invention provides a compound that binds IL-13 and TSLP for use in reducing the level of fractional exhaled nitric oxide (FeNO) in a subject, wherein said compound reduces the level of FeNO by at least 20 ppb, at least 30 ppb, or by at least 40 ppb compared to a control.

In some embodiments, the invention provides a method for reducing the level of fractional exhaled nitric oxide (FeNO) in a subject, wherein said compound reduces the level of FeNO by at least 18 ppb compared to a control. The embodiments and examples given above for compounds that binds IL-13 and TSLP for use in the treatment of a pulmonary disease, wherein said treatment reduces the level of fractional exhaled nitric oxide (FeNO) compared to a control, can be transferred to methods for reducing the level of fractional exhaled nitric oxide (FeNO) in a subject. This applies in particular to the reduction of FeNO level, time points, the nature of the compound, and the control. E.g., in some embodiments, the invention provides a method for reducing the level of fractional exhaled nitric oxide (FeNO) in a subject, wherein said compound reduces the level of FeNO by at least 20 ppb, at least 30 ppb, or by at least 40 ppb compared to a control.

### 5.5 Preventive reduction of FeNO level

In another aspect, the present invention relates to a preventive aspect. As the present invention provides ways to reduce FeNO level to a larger extent than known treatments, this opens the possibility of preventively treat patients with elevated FeNO level to prevent a loss of lung function before it even occurs. This preventive treatment can be administered to asthma patients and subjects having the risk of developing asthma as well as patients with other pulmonary diseases and subjects having the risk of developing other pulmonary diseases. Accordingly, the present invention also provides compounds for use in reducing the FeNO level in a subject and methods for reducing the FeNO level in a subject, wherein the reduction of FeNO level prevents a loss of lung function.

### FeNO level

The preventive reduction of FeNO level according to the present invention relates to patients with an elevated FeNO level before the start of the preventive treatment (termed baseline FeNO level hereafter). An elevated baseline FeNO level is a FeNO level which is above 25 ppb (see e.g. Miskoff et al.).

In some embodiments, the present invention provides compounds for use in reducing the FeNO level in a subject with a baseline FeNO level of at least 90 ppb, wherein the reduction of FeNO level prevents a loss of lung function. In some embodiments, the present invention provides compounds for use in reducing the FeNO level in a subject with a baseline FeNO level of at least 80 ppb, wherein the reduction of FeNO level prevents a loss of lung function. In some embodiments, the present invention provides compounds for use in reducing the FeNO level in a subject with a baseline FeNO level of at least 75 ppb, wherein the reduction of FeNO level prevents a loss of lung function. In some embodiments, the present invention provides compounds for use in reducing the FeNO level in a subject with a baseline FeNO level of at least 70 ppb, wherein the reduction of FeNO level prevents a loss of lung function. In some embodiments, the present invention provides compounds for use in reducing the FeNO level in a subject with a baseline FeNO level of at least 65 ppb, wherein the reduction of FeNO level prevents a loss of lung function. In some embodiments, the present invention provides compounds for use in reducing the FeNO level in a subject with a baseline FeNO level of at least 60 ppb, wherein the reduction of FeNO level prevents a loss of lung function. In some embodiments, the present invention provides compounds for use in reducing the FeNO level in a subject with a baseline FeNO level of at least 55 ppb, wherein the reduction of FeNO level prevents a loss of lung function. In some embodiments, the present invention provides compounds for use in reducing the FeNO level in a subject with a baseline FeNO level of at least 50 ppb, wherein the reduction of FeNO level prevents a loss of lung function. In some embodiments, the present invention provides compounds for use in reducing the FeNO level in a subject with a baseline FeNO level of at least 45 ppb, wherein the reduction of FeNO level prevents a loss of lung function. In some embodiments, the present invention provides compounds for use in reducing the FeNO level in a subject with a baseline FeNO level of at least 40 ppb, wherein the reduction of FeNO level prevents a loss of lung function. In some embodiments, the present invention provides compounds for use in reducing the FeNO level in a subject with a baseline FeNO level of at least 35 ppb, wherein the reduction of FeNO level prevents a loss of lung function. In some embodiments, the present invention provides compounds for use in reducing the FeNO level in a subject with a baseline FeNO level of at least 30 ppb, wherein the reduction of FeNO level prevents a loss of lung function. In some embodiments, the present invention provides compounds for use in reducing the FeNO level in a subject with a baseline FeNO level of at least 25 ppb, wherein the reduction of FeNO level prevents a loss of lung function.

### FeNO reduction

In some embodiments, said reduction of FeNO level is a reduction by at least 18 ppb. In some embodiments, said reduction of FeNO level is a reduction by at least 20 ppb. In some embodiments, said reduction of FeNO level is a reduction by at least 25 ppb. In some embodiments, said reduction of FeNO level is a reduction by at least 30 ppb. In some embodiments, said reduction of FeNO level is a reduction by at least 35 ppb. In some embodiments, said reduction of FeNO level is a reduction by at least 40 ppb. In some embodiments, said reduction of FeNO level is a reduction to a normal level which means reduction to a level of below 25 ppb.

### FeNO level + FeNO reduction

In some embodiments, the present invention provides compounds for use in reducing the FeNO level in a subject with a baseline FeNO level of at least 50 ppb, wherein the reduction of FeNO level prevents a loss of lung function and wherein said reduction of FeNO level is a reduction by at least 25 ppb. In some embodiments, the present invention provides compounds for use in reducing the FeNO level in a subject with a baseline FeNO level of at least 50 ppb, wherein the reduction of FeNO level prevents a loss of lung function and wherein said reduction of FeNO level is a reduction by at least 20 ppb. In some embodiments, the present invention provides compounds for use in reducing the FeNO level in a subject with a baseline FeNO level of at least 50 ppb, wherein the reduction of FeNO level prevents a loss of lung function and wherein said reduction of FeNO level is a reduction by at least 18 ppb. In some embodiments, the present invention provides compounds for use in reducing the FeNO level in a subject with a baseline FeNO level of at least 50 ppb, wherein the reduction of FeNO level prevents a loss of lung function and wherein said reduction of FeNO level is a reduction to a level of below 25 ppb.

In some embodiments, the present invention provides compounds for use in reducing the FeNO level in a subject with a baseline FeNO level of at least 35 ppb, wherein the reduction of FeNO level prevents a loss of lung function and wherein said reduction of FeNO level is a reduction by at least 25 ppb. In some embodiments, the present invention provides compounds for use in reducing the FeNO level in a subject with a baseline FeNO level of at least 35 ppb, wherein the reduction of FeNO level prevents a loss of lung function and wherein said reduction of FeNO level is a reduction by at least 20 ppb. In some embodiments, the present invention provides compounds for use in reducing the FeNO level in a subject with a baseline FeNO level of at least 35 ppb, wherein the reduction of FeNO level prevents a loss of lung function and wherein said reduction of FeNO level is a reduction by at least 18 ppb. In some embodiments, the present invention provides compounds for use in reducing the FeNO level in a subject with a baseline FeNO level of at least 35 ppb, wherein the reduction of FeNO level prevents a loss of lung function and wherein said reduction of FeNO level is a reduction to a level of below 25 ppb.

In some embodiments, the present invention provides compounds for use in reducing the FeNO level in a subject with a baseline FeNO level of at least 25 ppb, wherein the reduction of FeNO level prevents a loss of lung function and wherein said reduction of FeNO level is a reduction by at least 25 ppb. In some embodiments, the present invention provides compounds for use in reducing the FeNO level in a subject with a baseline FeNO level of at least 25 ppb, wherein the reduction of FeNO level prevents a loss of lung function and wherein said reduction of FeNO level is a reduction by at least 20 ppb. In some embodiments, the present invention provides compounds for use in reducing the FeNO level in a subject with a baseline FeNO level of at least 25 ppb, wherein the reduction of FeNO level prevents a loss of lung function and wherein said reduction of FeNO level is a reduction by at least 18 ppb. In some embodiments, the present invention provides compounds for use in reducing the FeNO level in a subject with a baseline FeNO level of at least 25 ppb, wherein the reduction of FeNO level prevents a loss of lung function and wherein said reduction of FeNO level is a reduction to a level of below 25 ppb.

### Compounds for use in the preventive reduction of FeNO level

In some embodiments, said compound is a compound which binds to IL-13. Exemplary compounds which bind Il-13 are the antibodies anrukinzumab, lebrikizumab and tralokinumab.

In some embodiments, said compound is a compound which binds to TSLP. An exemplary compound which binds TSLP is the antibody Tezepelumab.

In some embodiments, said compound is a compound which binds to IL-13 and TSLP. Exemplary compounds which bind IL-13 and TSLP are given above. (section 5.3). In some embodiments, said compound is a polypeptide which comprises or consists of the amino acid sequence of SEQ ID NO: 1.

### FeNO level + FeNO reduction + Compound

In some embodiments, the present invention provides compounds for use in reducing the FeNO level in a subject with a baseline FeNO level of at least 50 ppb, wherein the reduction of FeNO level prevents a loss of lung function, wherein said reduction of FeNO level is a reduction by at least 25 ppb, and wherein said compound is a compound which binds to IL-13 and TSLP. In some embodiments, the present invention provides compounds for use in reducing the FeNO level in a subject with a baseline FeNO level of at least 50 ppb, wherein the reduction of FeNO level prevents a loss of lung function, wherein said reduction of FeNO level is a reduction by at least 25 ppb, and wherein said compound is a polypeptide which comprises or consists of the amino acid sequence of SEQ ID NO: 1. In some embodiments, the present invention provides compounds for use in reducing the FeNO level in a subject with a baseline FeNO level of at least 50 ppb, wherein the reduction of FeNO level prevents a loss of lung function, wherein said reduction of FeNO level is a reduction by at least 25 ppb, and wherein said compound Tezepelumab.

In some embodiments, the present invention provides compounds for use in reducing the FeNO level in a subject with a baseline FeNO level of at least 50 ppb, wherein the reduction of FeNO level prevents a loss of lung function, wherein said reduction of FeNO level is a reduction to a level of below 25 ppb, and wherein said compound is a compound which binds to IL-13 and TSLP. In some embodiments, the present invention provides compounds for use in reducing the FeNO level in a subject with a baseline FeNO level of at least 50 ppb, wherein the reduction of FeNO level prevents a loss of lung function, wherein said reduction of FeNO level is a reduction to a level of below 25 ppb, and wherein said compound is a polypeptide which comprises or consists of the amino acid sequence of SEQ ID NO: 1. In some embodiments, the present invention provides compounds for use in reducing the FeNO level in a subject with a baseline FeNO level of at least 50 ppb, wherein the reduction of FeNO level prevents a loss of lung function, wherein said reduction of FeNO level is a reduction to a level of below 25 ppb, and wherein said compound Tezepelumab.

In some embodiments, the present invention provides compounds for use in reducing the FeNO level in a subject with a baseline FeNO level of at least 35 ppb, wherein the reduction of FeNO level prevents a loss of lung function, wherein said reduction of FeNO level is a reduction by at least 18 ppb, and wherein said compound is a compound which binds to IL-13 and TSLP. In some embodiments, the present invention provides compounds for use in reducing the FeNO level in a subject with a baseline FeNO level of at least 35 ppb, wherein the reduction of FeNO level prevents a loss of lung function, wherein said reduction of FeNO level is a reduction by at least 18 ppb, and wherein said compound is a polypeptide which comprises or consists of the amino acid sequence of SEQ ID NO: 1. In some embodiments, the present invention provides compounds for use in reducing the FeNO level in a subject with a baseline FeNO level of at least 35 ppb, wherein the reduction of FeNO level prevents a loss of lung function, wherein said reduction of FeNO level is a reduction by at least 18 ppb, and wherein said compound Tezepelumab.

In some embodiments, the present invention provides compounds for use in reducing the FeNO level in a subject with a baseline FeNO level of at least 35 ppb, wherein the reduction of FeNO level prevents a loss of lung function, wherein said reduction of FeNO level is a reduction to a level of below 25 ppb, and wherein said compound is a compound which binds to IL-13 and TSLP. In some embodiments, the present invention provides compounds for use in reducing the FeNO level in a subject with a baseline FeNO level of at least 35 ppb, wherein the reduction of FeNO level prevents a loss of lung function, wherein said reduction of FeNO level is a reduction to a level of below 25 ppb, and wherein said compound is a polypeptide which comprises or consists of the amino acid sequence of SEQ ID NO: 1. In some embodiments, the present invention provides compounds for use in reducing the FeNO level in a subject with a baseline FeNO level of at least 35 ppb, wherein the reduction of FeNO level prevents a loss of lung function, wherein said reduction of FeNO level is a reduction to a level of below 25 ppb, and wherein said compound Tezepelumab.

In some embodiments, the present invention provides compounds for use in reducing the FeNO level in a subject with a baseline FeNO level of at least 25 ppb, wherein the reduction of FeNO level prevents a loss of lung function, wherein said reduction of FeNO level is a reduction by at least 18 ppb, and wherein said compound is a compound which binds to IL-13 and TSLP. In some embodiments, the present invention provides compounds for use in reducing the FeNO level in a subject with a baseline FeNO level of at least 25 ppb, wherein the reduction of FeNO level prevents a loss of lung function, wherein said reduction of FeNO level is a reduction by at least 18 ppb, and wherein said compound is a polypeptide which comprises or consists of the amino acid sequence of SEQ ID NO: 1. In some embodiments, the present invention provides compounds for use in reducing the FeNO level in a subject with a baseline FeNO level of at least 25 ppb, wherein the reduction of FeNO level prevents a loss of lung function, wherein said reduction of FeNO level is a reduction by at least 18 ppb, and wherein said compound Tezepelumab.

### Cause of loss of lung function

Elevated FeNO level is linked to multiple lung diseases, including asthma or idiopathic pulmonary fibrosis (Cameli et al.). Subjects with an elevated baseline FeNO level can either already show symptoms of asthma of another lung disease or are at risk of developing symptoms of asthma or another lung disease. Amongst those symptoms is loss of lung function. This loss of lung function can be prevented by the preventive reduction of FeNO level according to the present invention.

In some embodiments, the reduction of FeNO level according to the present invention prevents a loss of lung function linked to asthma. In some embodiments, the reduction of FeNO level according to the present invention prevents a loss of lung function linked to a lung disease which is not asthma.

FeNO level + FeNO reduction + Compound + Cause of loss of lung function

In some embodiments, the present invention provides compounds for use in reducing the FeNO level in a subject with a baseline FeNO level of at least 50 ppb, wherein the reduction of FeNO level prevents a loss of lung function, wherein said reduction of FeNO level is a reduction to a level of below 25 ppb, wherein said compound is a compound which binds to IL-13 and TSLP, and wherein said a loss of lung function is linked to asthma. In some embodiments, the present invention provides compounds for use in reducing the FeNO level in a subject with a baseline FeNO level of at least 50 ppb, wherein the reduction of FeNO level prevents a loss of lung function, wherein said reduction of FeNO level is a reduction to a level of below 25 ppb, wherein said compound is a polypeptide which comprises or consists of the amino acid sequence of SEQ ID NO: 1, and wherein said a loss of lung function is linked to asthma. In some embodiments, the present invention provides compounds for use in reducing the FeNO level in a subject with a baseline FeNO level of at least 50 ppb, wherein the reduction of FeNO level prevents a loss of lung function, wherein said reduction of FeNO level is a reduction to a level of below 25 ppb, wherein said compound Tezepelumab, and wherein said a loss of lung function is linked to asthma.

In some embodiments, the present invention provides compounds for use in reducing the FeNO level in a subject with a baseline FeNO level of at least 50 ppb, wherein the reduction of FeNO level prevents a loss of lung function, wherein said reduction of FeNO level is a reduction to a level of below 25 ppb, wherein said compound is a compound which binds to IL-13 and TSLP, and wherein said loss of lung function is linked to a lung disease which is not asthma. In some embodiments, the present invention provides compounds for use in reducing the FeNO level in a subject with a baseline FeNO level of at least 50 ppb, wherein the reduction of FeNO level prevents a loss of lung function, wherein said reduction of FeNO level is a reduction to a level of below 25 ppb, wherein said compound is a polypeptide which comprises or consists of the amino acid sequence of SEQ ID NO: 1, and wherein said loss of lung function is linked to a lung disease which is not asthma. In some embodiments, the present invention provides compounds for use in reducing the FeNO level in a subject with a baseline FeNO level of at least 50 ppb, wherein the reduction of FeNO level prevents a loss of lung function, wherein said reduction of FeNO level is a reduction to a level of below 25 ppb, wherein said compound Tezepelumab, and wherein said loss of lung function is linked to a lung disease which is not asthma.

The present invention also provides methods for reducing the FeNO level in a subject, wherein the reduction of FeNO level prevents a loss of lung function. All embodiments and examples given above for compounds for use in reducing the FeNO level in a subject, wherein the reduction of FeNO level prevents a loss of lung function can be transferred to methods for reducing the FeNO level in a subject, wherein the reduction of FeNO level prevents a loss of lung function.

### 5.6 Other aspects of the present invention

### Treatment of small airway obstruction

The present invention also provides compounds for use in the treatment of a pulmonary disease, wherein said treatment reduces small airway obstruction. Thus, the present invention provides the possibility to specifically treat patients who suffer from small airway obstruction, e.g. the subgroup of asthma patients who suffer from small airway obstruction.

Accordingly, the present invention provides a compound that binds IL-13 and TSLP for use in the treatment of a pulmonary disease, wherein said treatment reduces the level of fractional exhaled nitric oxide (FeNO) by at least 18 ppb compared to a control, wherein said treatment reduces small airway obstruction. In some embodiments, the pulmonary disease is asthma. In some embodiments, the control is placebo. In some embodiments, the compound is a polypeptide which comprises or consists of the amino acid sequence of SEQ ID NO: 1. In some embodiments, pulmonary disease is asthma and the control is placebo. In some embodiments, the pulmonary disease is asthma and the compound is a polypeptide which comprises or consists of the amino acid sequence of SEQ ID NO: 1. In some embodiments, the control is placebo and the compound is a polypeptide which comprises or consists of the amino acid sequence of SEQ ID NO: 1. In some embodiments, the pulmonary disease is asthma, the control is placebo and the compound is a polypeptide which comprises or consists of the amino acid sequence of SEQ ID NO: 1.

### Reduction of eosinophil count

The present invention also provides compounds for use in reducing the eosinophil count in blood in a subject and methods for reducing the eosinophil count in blood in a subject.

In some embodiments, the invention provides a compound that binds IL-13 and TSLP for use in reducing the level of fractional exhaled nitric oxide (FeNO) in a subject, wherein said treatment reduces the eosinophil count in blood by at least 30% compared to a control. The embodiments and examples given above for compounds that binds IL-13 and TSLP for use in the treatment of a pulmonary disease, wherein said treatment reduces the eosinophil count in blood by at least 30% compared to a control, can be transferred to compounds that binds IL-13 and TSLP for use in reducing the eosinophil count in a subject. This applies in particular to reduction of eosinophil count, time points, the nature of the compound, and the control. E.g., in some embodiments, the invention provides a compound that binds IL-13 and TSLP for use in reducing the level of fractional exhaled nitric oxide (FeNO) in a subject, wherein said treatment reduces the eosinophil count in blood by at least 35% or by at least 40% compared to a control.

In some embodiments, the invention provides a method for reducing the eosinophil count in a subject, wherein said treatment reduces the eosinophil count in blood by at least 30% compared to a control. The embodiments and examples given above for compounds that binds IL-13 and TSLP for use in the treatment of a pulmonary disease, wherein said treatment reduces the eosinophil count in blood compared to a control, can be transferred to methods for reducing the eosinophil count in a subject. This applies in particular to reduction of eosinophil count, time points, the nature of the compound, and the control. E.g., in some embodiments, the invention provides a method for reducing the eosinophil count in a subject, wherein said treatment reduces the eosinophil count in blood by at least 35% or by at least 40% compared to a control.

### Reduction of the proportion of non-classical monocytes

The present invention also provides compounds for use in reducing the proportion of non-classical monocytes in the respiratory tract of a subject and methods for reducing the proportion of non-classical monocytes in the respiratory tract of a subject.

Monocytes are a heterogenous cell population with potential to become activated, infiltrate tissue from blood, and differentiate into macrophages. When activated, monocytes and macrophages release a number of inflammatory cytokines implicated in asthma pathogenesis, and monocyte activation and respiratory tissue infiltration have been described in asthma in numerous studies, suggesting an important role for this cell type in disease pathobiology (Li et al., Tomita et al.). Monocytes have been observed to rapidly accumulate in the nasal mucosa after local allergen challenge, where they promote recruitment of Th2 cells and eosinophils. A recent analysis of bronchoalveolar lavage fluid by single cell RNAseq has further identified several monocyte clusters, and a monocyte-derived macrophage subpopulation as being increased in patients with asthma exacerbations. Another recent study found monocytes accumulated in the lungs of children and adolescents with fatal asthma attacks (Eguiluz-Gracia et al.), results which also strongly support the direct involvement of monocytes in the immunopathology of asthma as pro-inflammatory cells and suggest that prevention of their entry into respiratory tissue by SAR might have beneficial effects in preventing asthma symptoms and exacerbations.

Therefore, the reduction of the proportion of a subpopulation of monocytes, e.g. non-classical monocytes, can be useful for asthma treatment. The present invention can achieve such a reduction, as shown below in the example section.

### Reduction of the proportion of NK cells

The present invention also provides compounds for use in reducing the proportion of NK cells in the blood of a subject and methods for reducing the proportion of NK cells in the blood of a subject.

### 5.7 Industrial applicability

The compound for use according to the present invention may be used in the treatment of subjects suffering from pulmonary diseases, such as asthma.

### 6 Examples

### 6.1 Example 1: PDY16622 - A double-blind, randomized, placebo-controlled, parallel design, single dose asthma study in three centers.

The efficacy of SAR443765 for the treatment of asthma was tested in a phase I trial (PDY16622) detailed below. The schedule of the trial is depicted in Fig. 1.

### Methods

### Patient cohort

A total of up to 36 participants with mild-to-moderate asthma was enrolled for this study. The participants were randomized in SAR443765 and placebo groups (24 SAR443765 and 12 placebo). The participants received a single dose at the highest safe and well tolerated dose level as assessed earlier in a study part with healthy adult participants (400 mg SC max).

The participants had to fulfil the following criteria:
- Diagnosis of asthma for at least 12 months and confirmed at screening based on the Global Initiative for Asthma (GINA) 2020 Guidelines, all steps except Step 4 with high inhaled corticosteroid (ICS) dose and Step 5.
- Controlled asthma defined as an Asthma Control Questionnaire (ACQ)-5 score of <1.5.
- Participants using as-needed short acting beta-agonist (SABA), ICS-naïve or with existing stable treatment (at least for 3 months prior to screening) with low to medium daily dose ICS (≤500 mcg of fluticasone propionate or comparable ICS total daily dosage) potentially in combination with a long-acting beta-agonist (LABA) and/or long acting muscarinic agonist (LAMA) as second controller, and/or with stable daily leukotriene receptor antagonist, leukotriene synthesis inhibitor and/or chromones.
- Participants with elevated FeNO level defined as ≥25 ppb at screening and baseline.
- Participants with prebronchodilator FEV1 ≥60% of predicted normal at screening.
- Reversibility of at least 12% and 200 mL in FEV1 or forced vital capacity (FVC) after administration of 4 puffs (400 mcg) of albuterol/salbutamol or levalbuterol/levosalbutamol during screening or documented history of a reversibility test that meets these criteria within 5 years prior to screening or documented positive response to methacholine challenge (a decrease in FEV1 by 20% [PC20] of <8 mg/mL) within 5 years prior to screening visit.
- Normal or clinically acceptable vital signs (pulse rate, SBP and DBP) after 10 minutes resting in supine position at screening and at baseline.
- Normal or clinically acceptable standard 12-lead electrocardiogram (ECG) parameters and tracing after 10 minutes resting in supine position at screening and at baseline; normal ECG tracing unless the Investigator considers an ECG tracing abnormality to be not clinically relevant.
- Laboratory parameters within the normal range (or defined screening threshold for the Investigator site) at screening and at baseline, unless the Investigator considers an abnormality to be clinically irrelevant; however, serum creatinine, alkaline phosphatase, hepatic enzymes (aspartate aminotransferase, alanine aminotransferase) should not exceed 1.25-fold the upper laboratory norm. Total bilirubin out of normal range can be acceptable if total bilirubin does not exceed 1.5-fold the upper limit with normal conjugated bilirubin values (unless the participant has documented Gilbert syndrome).
- Body weight between 50.0 kg and 105.0 kg, inclusive, if male, and between 40.0 kg and 95.0 kg, inclusive, if female, body mass index between 18.0 kg/m² and 32.0 kg/m², inclusive, at screening and at baseline.

### Dosing

Participants were dosed in subgroups of maximum 4 participants, at least 10 minutes apart, with a dosing interval of at least 2 days between subgroups. Initiation of the Asthma cohort was based on the blinded safety and PK data of an earlier study part with healthy adult participants.

All participants were screened within 28 days to ensure that they meet the study requirements. Eligible participants were admitted on the day prior to each dosing and participants included in the study remained institutionalized for 1 day after study drug administration in the Asthma cohort, taking into account emerging safety, PK and PD data. Prior to each drug administration, participants were assessed for SARS-CoV-2 infection and underwent intensive monitoring including physical examination, AE assessment, vital signs, ECGs, and routine laboratory assessment (complete blood count with differential, chemistry, aPTT, PT-INR, high-sensitivity cardiac troponin T and urinalysis). After institutionalization period(s), participants underwent clinical assessment including vital signs, ECG, routine laboratory assessment, and ADA assessment at varying intervals until study completion. Taking into account the range for half-life of serum albumin-binding nanobodies, the treatment emergent adverse events observation period for this study was therefore ended 70 days after the last dose.

### Fractional Exhaled Nitric Oxide (FeNO) level analysis

Fraction of exhaled nitric oxide (FeNO) was measured at the time points specified in the schedule of the study (Fig. 1), i.e. baseline, D8, D15, D29, and D57. FeNO is a measure of NO production by epithelial cells in the lung and considered a biomarker for airway inflammation in asthma.

FeNO levels (ppb) were collected on site with a dedicated medical device such as a commercially available hand worn device (NIOX VERO^{®}). The FeNO test needs to be completed prior to impulse oscillometry and spirometry in order to avoid any impact on the nitric oxide measurement. Considering the diurnal variation in FeNO, the assessment needs to be performed at approximately the same time of day throughout the study (±2 hours). Participants should not eat or drink 1 hour prior to FeNO measurement, as this may affect the results. All carbonated drinks and nitrate rich foods (e.g., vegetable juices, salads, lettuce, radishes, celery, broccoli, cauliflower, spinach, rocket, beets, parsley, leeks, cabbage, fennel, turnips, carrots, cured meats, sausage, bacon) should be withheld for at least 2 hours prior to the FeNO measurement. ICS, if any, should be withheld for at least 4 hours prior to FeNO measurement. The participant should be sitting during FeNO testing; however, if the participant is unable to sit, then standing is acceptable. The position (sitting or standing) should remain the same for a participant throughout the study. Participants are to inhale to total lung capacity through the hand worn device (NIOX VERO^{®}) and then exhale for 10 seconds at 50 mL/sec (assisted by visual and auditory cues).

### Eosinophil count

The eosinophil count in whole blood was determined at the time points specified in the schedule of the study (Fig. 1), i.e. at baseline and at D2 (24 h), D4 (72 h) D8 (1 week), D15 (2 weeks), D29 (4 weeks), D57 (8 weeks), and D71 (10 weeks) using flow cytometry.

### Spirometry measurements (FEV1 and FEF25-75)

FEV1 and FEF25-75 were determined by spirometry at the time points specified in the schedule of the study (Fig. 1).

Spirometry was performed in accordance with the American Thoracic Society (ATS)/European Respiratory Society (ERS) guidelines (2019 update). For the measured parameters, including FEV1, peak exploratory flow (PEF), FVC and forced exploratory flow (FEF) 25%-75%, spirometry was performed after a wash out period of bronchodilators according to their action duration as detailed in the ATS guidelines. For example, bronchodilator withholding time is at least 6 hours for SABA, at least 24 hours for LABA and 36-48 hours for LAMA. Chromones need to be withheld for at least 24 hours before spirometry. ICS, leukotriene receptor antagonists and leukotriene synthesis inhibitors need not to be withheld prior to spirometry, however ICS need to be withheld prior to FeNO assessment.

At all visits, spirometry was performed preferably in the morning, afternoon was allowable in the exceptional circumstance when morning spirometry could not be performed. Spirometry was done at approximately the same time at each visit throughout the study (±2 hours). The same spirometer and standard spirometric techniques, including calibration, was used to perform spirometry at all visits, and whenever possible, the same person performed the measurements. Three measurements fulfilling the ATS acceptability and repeatability criteria should be obtained at every visit.

Reversibility was determined by a postbronchodilator spirometry measurement. Reversibility is defined as an increase of the absolute FEV1 and/or FVC after administration of bronchodilator and is measured by spirometry as postbronchodilator increase in FEV1 or FVC in percent of the prebronchodilator FEV1 or FVC, respectively. After spirometry for measuring prebronchodilator FEV1, participants received 4 puffs of albuterol/salbutamol or levalbuterol/levosalbutamol from a primed metered dose inhaler (MDI). The postbronchodilator spirometry should be performed after a waiting time of at least 10 minutes and may be repeated several times within approximately 30 minutes after administration of bronchodilator.

Impulse oscillometry measurements (R5-20 and AX)

R5-20 and AX were determined by oscillometry at the time points specified in the schedule of the study (Fig. 1).

Oscillometry is a complementary technique to spirometry determining the mechanic properties of the lung. Whereas spirometry is the most commonly used technique examining airway function, it is unable to sensitively evaluate small airways, becoming abnormal only when approximately 75% of small airways are obstructed. Oscillometry is more sensitive in detecting small airway disease, which correlates with poor disease control and type 2 inflammation. Thus, oscillometry allows to assess the relative contribution of the large and small airways in asthma. Oscillometry was conducted during tidal breathing using the Tremoflo^{®} device (Thorasys, Montreal, Canada) according to ERS recommended guidelines (Oostveen et al., 2003). Oscillometry was performed immediately prior to spirometry according to the study protocol.

A multi-frequency signal from 5 to 37 Hz super-imposed oscillatory pressure and flow on the participant's spontaneous breathing. Measurements of 20 sec were repeated with breaks of about 20 sec. Artefacts due to cough or glottis closure were removed by automatic rejection by the software. A minimum of 3 recordings that achieved coefficient of variation of ≤15% was required for quality control (Peters et al., Appl Physiol Nutr Metab. 2016;41(5):538-47). Only these data were used for the data analysis.

Low frequencies (e.g. at 5Hz) reach the small airways and reflect the total airway whereas high frequencies (e.g. at 20 Hz) do not reach the small airways and therefore reflect only the central airways. Respiratory resistance (Rrs) reflects the energy needed to propagate the pressure wave through the airways and distend the lunch parenchyma. Total airway resistance is determined predominantly by the central airways (80%) and to a lesser extent by the smaller airways (20%). As such, in health there is a low frequency dependence of Rrs (Pride, Thorax. 1992;47(4):317-20) and R5-20 (the difference between Rrs at 5Hz (R5) and at 20Hz (R20)) is low. Central airway flow obstruction, a component of asthmatic airflow limitation, increases Rrs at all frequencies (i.e. both R5 and R20 are increased) and R5-20 is low, (Làndsér et al., Chest. 1982;81(5):586-91). In small airway disease, also frequently seen in asthma, Rrs increase in a frequency-dependent fashion at low frequencies (i.e. R5 increases more than R20) . (Clément et al., Chest. 1983;83(2):215-20). Thus, the frequency dependence of Rrs (called R5-20; the difference between Rrs at 5Hz and 20Hz) reflects airway heterogeneity and increases with small airway obstruction (Otis et al., J Appl Physiol. 1956;8(4):427-43).

Respiratory reactance (Xrs) is driven by the capacitance (recoil) properties of the respiratory system at low frequencies where it reflects the soft tissue and lung parenchyma. At higher frequencies, however, Xrs is driven by the inertia of the moving air column in the conducting airways. The resonant frequency (fres) is the point at which the magnitudes of capacitive and inertive reactance are equal; fres is increased in both obstructive and restrictive lung diseases (Pride, Thorax. 1992;47(4):317-20, Clément et al., Chest. 1983;83(2):215-20). AX is an integrative index of total respiratory reactance at all frequencies between 5 Hz and fres (area under the reactance curve) and has the units of elastance, and is a measurement of small airways disease and closure, as seen in asthma.

R5, R5-20, and AX are the most sensitive oscillometry metrics of small airway function (Goldman et al., Oostveen et al., 2003, Oostveen et al. 2013). Thus, AX, R5, and R5-20 were the focus of the analysis for detection of treatment effect.

### Transcriptomic analysis

To enhance the understanding of the effect of SAR443765 on a cellular level, single-cell RNA sequencing (scRNA-seq) was utilized. This technique allows studying gene expression patterns at the individual cell level, enabling a more detailed and comprehensive analysis. By comparing the gene expression patterns before and after SAR443765 treatment, as well as SAR443765 treatment versus the placebo group, specific cellular changes linked to SAR443765 treatment can be identified.

In the present study, scRNAseq was performed for nasal brushing (NB) samples and peripheral blood leukocyte (PBL) samples, taken at D1 (before administration of SAR443765 or placebo) and D29 from the SAR443765 group as well as the placebo group.
scRNAseq was performed as described in the literature (see e.g. Haque et al. and Slovin et al.). Analyses were performed separately for Nasal Brushing and PBL data. Results for cell populations and gene expression from different samples were compared as a follow-up analysis to assess consistency across specimens.

SignacX v 2.2.4 was used to annotate cell types B.memory, B.naive, Macrophages, Mon.Classical, Mon.NonClassical, Neutrophils, Eosinophil, NK, Plasma, CD4.T.memory, CD4.T.naive, CD8.T.CM, CD8.T.EM, CD8.T.naive, Tregs, Fibroblasts, Endothelial, and Epithelial cell types (Chamberlain et al.). As SignacX provides detailed algorithmic annotation of multiple immune types but only a general classification for non-immune cell types, Louvain clustering run during SPRING preprocessing followed by marker gene expression analysis was applied for annotation of NB non-immune epithelial cell subtypes, dendritic cell subtypes (Villani et al.), and mast and eosinophil cells. Manual annotation of nasal epithelium was performed based on cluster-level expression of previously published marker sets and marker gene set ranked expression (Deprez et al., Legebeke et al., Vieira Braga et al.). SPRING pipeline annotated objects were converted to common scRNAseq format data structures (Seurat and SingleCellExperiment objects) which were used as inputs to other single cell data analysis packages for further analysis.

To summarize the results of pre-processing and annotation, with the normalized count data dimensionality reduction, clustering and cell-type annotation were performed including identification of novel cell types and subtypes. Summary outputs showed the distribution of cell type abundance in terms of proportion and percentages, top markers genes per cell type were reported to confirm assignments. These outputs were presented graphically.

Samples collected at baseline were analyzed to assess the presence of cell subsets and/or gene signatures potentially predictive of reduction in FeNO. FeNO values at week 4 (D29) and/or changes in FeNO from baseline were used for correlation analyses. In particular, cell types that emerged as prominent in the scRNASeq analyses were subjected to spearman correlation analysis with FeNO values at week 4 (D29) and/or changes in FeNO between D29 and D1 (baseline). Various correlation analyses were performed such as change in cell types proportions from baseline vs change in FeNO, gene expression at baseline vs change in FeNO. Correlation values along with p-values were reported for these analyses.

### Results

The following data were collected at the time points indicated in Fig. 1: FeNO levels (Fig. 2 and 3); eosinophil count in blood (Fig. 4 and 5), FEV1 (Fig. 6,7, 8), FEF25-75 (Fig. 9), R5-20 (Fig. 10), AX (Fig. 11), other biomarkers in serum/plasma (Fig. 12), transcriptomic analysis (Fig. 13, 14, 15, 16).

### FeNO level

Fig. 2 shows the change from baseline in FeNO level of participants which have been treated with SAR443765 (dashed line) or placebo (solid line). The dose of SAR443765 was 400 mg, FeNO levels were measured one week (D8), two weeks (D15), 4 weeks (D29), and 8 weeks (D57) after the administration of SAR443765 or placebo. Table 1 shows the results of those FeNO level measurements. The baseline value is defined as the last available value before and closest to the first dose of investigational medicinal product. SAR443765 treatment was determined to reduce the FeNO level by 31.1 ppb (-31.9 ppb - -0.8 ppb) after one week (D8), by 54.0 ppb (-35.2 ppb - 18.8 ppb) after 2 weeks (D15), by 39.9 ppb (-39.1 ppb - 0.8 ppb) after 4 weeks (D29), and by 35.9 ppb (-32.0 ppb - 3.9 ppb) after 8 weeks (D57) compared to placebo.

In the SAR443765 group, 37% of the participants had a normal FeNO level (below 25 ppb) at the end of the study, while this was only the case for 8% of the placebo group.

**Table 1: Change in FeNO levels after SAR443765 treatment. All FeNO levels are indicated in ppb.**

| Treatment group / time point | FeNO level | | | Change from baseline | | |
|---|---|---|---|---|---|---|
| | Mean | Standard Dev. | Median | Mean | Standard Dev. | Median |
| Placebo | | | | | | |
| Baseline | 71.9 | 44.8 | 54.5 | - | - | - |
| D8 | 71.1 | 50.2 | 49.5 | -0.8 | 29.6 | 0.5 |
| D15 | 90.7 | 70.6 | 69.5 | 18.8 | 38.9 | 11.5 |
| D29 | 72.8 | 54.5 | 48.5 | 0.8 | 28.8 | 2.0 |
| D57 | 75.8 | 46.4 | 73.5 | 3.9 | 24.3 | 6.5 |

| SAR443765 (400 mg) | | | | | | |
|---|---|---|---|---|---|---|
| Baseline | 67.9 | 36.1 | 60.5 | - | - | - |
| D8 | 34.1 | 16.7 | 30.0 | -31.9 | 24.9 | -25.0 |
| D15 | 32.7 | 15.9 | 31.5 | -35.2 | 29.1 | -27.0 |
| D29 | 28.8 | 10.9 | 26.0 | -39.1 | 29.6 | -29.5 |
| D57 | 35.9 | 21.5 | 29.5 | -32.0 | 25.3 | -25.5 |

Table 2 presents the baseline FeNO values as well as the reduction after 4 weeks (D29) of the present study and multiple published results of studies with other biologics, including tezepelumab (anti-TSLP), lebrikizumab (anti-IL-13), tralokinumab (anti-IL-13), benralizumab (anti-IL-5Ra), dupilumab (anti-IL4Ra) and itepekimab (anti-IL-33). SAR443765 shows a stronger reduction of FeNO levels than all other compounds, with a more than 2-fold higher reduction compared to the highest reduction reached in a trial with another compound (18 ppb in a lebrikizumab trial). The present trial shows that the administration of a compound which blocks both IL-13 and TSLP can massively increase the FeNO reduction by a factor of approximately 2 to 4 compared to monospecific approaches.

**Table 2: Comparison of SAR443765 to other biologics used for asthma treatment. All FeNO levels are indicated in ppb.**

| Treatment arms | Baseline (standard deviation) | Change from baseline D29 | Difference drug vs. placebo | Reference |
|---|---|---|---|---|
| **SAR443765** (N=24) | 67.9 (36.1) | -39.1 | **-39.9** | Present invention |
| Placebo (N=12) | 71.9 (44.8) | 0.8 | | |
| Published results | | | | |
| **Tezepelumab** (N=15) | 58.9(55) | -18.1 | **-12.6** | Gauvreau, 2014 |
| Placebo (N=16) | 42.3 (17) | -5.5 | | |
| **Tezepelumab** (N=112) | 31.5 (39.8) | -8.5 | **-8** | Corren, 2017 |
| Placebo (N=119) | 37.8 (39.7) | -0.5 | | |
| **Tezepelumab** (N=491) | 41.4 (36.3) | -15 | **-12** | Menzies-Gow, 2021 |
| Placebo (N=491) | 46.3 (44.7) | -3 | | |
| **Tezepelumab** (N=69) | 38.7 (40.8) | -11.7 | **-10.3** | Wechsler, 2022 |
| Placebo (N=68) | 42.3 (37.4) | -1.4 | | |
| **Lebrikizumab** (N=104) | 34 (6-242) | -28 | **-18** | Korenblat, 2018 |
| Placebo (N=105) | 37 (6-232) | -10 | | |
| **Lebrikizumab** (N=107) | 31.0 (24.6) | -11.8 | **-8.3** | Corren, 2011 |
| Placebo (N=112) | 30.2 (27.7) | -3.5 | | |
| **Tralokinumab** (N=39) | 39.54 (30,05) | -14.2 | **-11.7** | Russell, 2018 |
| Placebo (N=40) | 32.23 (24,82) | -2.5 | | |
| **Tralokinumab** (N=363) | 29.8 (29.7) | ≈-7.0 | **-6.5** | Panettieri, 2018 |
| Placebo (N=400) | 29.7(28.5) | ≈-0.5 | | |
| **Benralizumab** (N=81) | 37.8 (31.7) | -4 | **-5** | Castro, 2014 |
| Placebo (N=80) | 39.9 (31.9) | +1 | | |
| **Dupilumab** (N=157) | 38.13 (35.11) | -16 | **-11** | Wenzel, 2016 |
| Placebo (N=158) | 38.95 (34.79) | -5 | | |
| **Itepekimab** (N=73) | 30.3 (24.3) | -7 | **-9** | Wechsler, 2021 |
| Placebo (N=74) | 33.3 (42.6) | 2 | | |

### Subgroup analysis

Fig. 3 shows the same measurements as Fig. 2 separated according to the eosinophil count baseline of the participants. The following groups are depicted: SAR443765 with high baseline eosinophil count (≥ 0.3 ^{∗}10⁹ cells/L (lower dashed line), SAR443765 with low baseline eosinophil count (< 0.3 ^{∗}10⁹ cells/L (lower solid line), placebo with high baseline eosinophil count (≥ 0.3 ^{∗}10⁹ cells/L (upper dashed line), placebo with low baseline eosinophil count (< 0.3 ^{∗}10⁹ cells/L (upper solid line). The results of those four groups at D29 (4 weeks) are also depicted in Table 3.

**Table 3: Change in FeNO levels after SAR443765 treatment for high eosinophilic and low eosinophilic subgroups. All FeNO levels are indicated in ppb.**

| Treatment group | Baseline FeND Mean (Standard deviation) | Change from baseline D29 (Standard deviation) | Percent change from baseline D29 (Standard deviation) |
|---|---|---|---|
| Placebo with EOS <0.3 (N=8) | 56.1 (38.7) | 1.4 (13.9) | -1.87 (23.46) |
| Placebo with EOS ≥0.3 (N=4) | 103.5 (43.3) | -0.3 (50.9) | 3.94 (47.83) |
| SAR443765 with EOS <0.3 (N=16) | 59.3 (35.9) | -32.1 (30.1) | -49.45 (21.24) |
| SAR443765 with EOS ≥0.3 (N=8) | 85.1 (31.8) | -53.3 (24.3) | -60.40 (9.81) |

The present treatment led to a remarkable reduction of FeNO level is both groups. For participants with high eosinophil count, the reduction compared to placebo is 53.0 ppb (64.34%of baseline), for participants with low eosinophil count, the reduction compared to placebo is 33.5 ppb (47.58% of baseline). This shows that the present treatment lead in both participant groups to similar relative FeNO reduction which exceed the FeNO reductions seen for any other biologic (see Table 2).

### Eosinophil count

Fig. 4 shows the change in eosinophil count of participants which have been treated with SAR443765 (right column) or placebo (left column) at D29 (4 weeks). The present treatment led to a median change in eosinophil count of -42.42 %, while the placebo group showed a median change in eosinophil count of-0.38%. This demonstrated that SAR443765 led to a strong reduction in eosinophil count compared to placebo. Fig. 5 shows the change in eosinophil count at D29 for SAR443765 and other biologics (lebrikizumab, tezepelumab and dupilumab). SAR443765 shows a decrease in the same range as tezepelumab. Note that dupilumab, which like SAR443765 blocks IL-13 signaling, shows here an increase in eosinophil count. Such an eosinophil increase was not seen for SAR443765.

Table 4 shows the results of eosinophil counts after SAR443765 treatment at D2, D4, D8, D15, D29, D57 and D71.

**Table 4: Change in eosinophil count after SAR443765 treatment. All eosinophil counts are indicated in 10⁹ cells/L.**

| Treatment group / time point | Eosinophil count | | | Percent change from baseline | | |
|---|---|---|---|---|---|---|
| | Mean | Standard Dev. | Median | Median | Min | Max |
| Placebo | | | | | | |
| Baseline | 0.280 | 0.119 | 0.275 | - | - | - |
| D2 (24 h) | 0.256 | 0.087 | 0.235 | -12.90 | -26.1 | 37.5 |
| D4 (72 h) | 0.259 | 0.172 | 0.195 | -4.85 | -46.9 | 30.5 |
| D8 | 0.225 | 0.181 | 0.225 | 3.13 | -43.5 | 42.9 |
| D15 | 0.395 | 0.183 | 0.395 | 38.39 | -48.1 | 168.8 |
| D29 | 0.255 | 0.238 | 0.255 | -0.38 | -33.3 | 306.3 |
| D57 | 0.307 | 0.158 | 0.290 | -4.82 | -50.0 | 156.3 |
| D71 | 0.299 | 0.185 | 0.245 | -14.78 | -56.0 | 150.0 |
| SAR443765 (400 mg) | | | | | | |
| Baseline | 0.281 | 0.143 | 0.250 | - | - | - |
| D2 (24 h) | 0.247 | 0.143 | 0.220 | -20.00 | -40.0 | 71.4 |
| D4 (72 h) | 0.180 | 0.088 | 0.170 | -34.67 | -77.8 | 40.7 |
| D8 | 0.194 | 0.141 | 0.140 | -40.83 | -77.8 | 318.8 |
| D15 | 0.186 | 0.095 | 0.165 | -30.91 | -70.6 | 137.5 |
| D29 | 0.161 | 0.119 | 0.150 | -42.42 | -100.0 | 268.8 |
| D57 | 0.215 | 0.309 | 0.140 | -46.25 | -83.8 | 263.6 |
| D71 | 0.180 | 0.159 | 0.115 | -51.00 | -80.0 | 143.8 |

### FEV1

FEV1 (forced expiratory volume in one second) is the maximal air volume which can be exhaled in the first second of expiration, starting from maximal inspiration. In this study, FEV1 was determined by spirometry as described above. Fig. 6 shows the change (compared to baseline) of FEV1 of participants which have been treated with SAR443765 (dashed line) or placebo (solid line). The dose of SAR443765 was 400 mg. Fig. 6 depicts all available FEV1 measurements. Fig. 7 shows the same measurements, but excludes values where the measurement did not meet all quality standards, meaning the difference between the 2 largest FEV1 values in a triplicate was >0.150 L. Taking the results from Fig. 6, the present treatment led to an increase in FEV1 of ca. 0.25 L after one week (D8), ca. 0.2 L after 2 weeks (D15), ca. 0.07 L after 4 weeks (D29) and 8 weeks (D57) compared to placebo. The results from Fig. 7 are similar for D8 and D29 but show a smaller difference for D15 and no meaningful difference at D57. Those results indicate that SAR443765 improves FEV1. However, further studies with a larger patient cohort and more impaired FEB1 at baseline are needed to corroborate this finding.

To get more insight in different patient subpopulations, the FEV1 improvement was analyzed for subpopulations with normal and impaired lung function at baseline. The criterion to classify a participants in one of those group was the "percent predicted FEV1" at baseline, i.e. before the start of the treatment with SAR443765 or placebo. The percent predicted FEV1 (abbreviated as "ppFEV1") is the ratio (in %) between the actual FEV1 of the participant and a reference FEV1 which reflects the average value for a person with the participant's demographics, like age, sex, and body composition. Participants whose baseline ppFEV1 was ≥80 % were classified in the normal baseline lung function subpopulation, while participants whose baseline ppFEV1 was <80% were classified in the impaired baseline lung function subpopulation. So the data for the SAR443765 group and the placebo group were both divided into two subpopulations according to this criterion. The FEV1 results for those 4 subgroups are shown in Fig. 8. The results indicate that the overall improvement of FEV1 seen in Fig. 7, especially for D8, D15, and D29 after SAR443765 treatment is mainly due to an improvement in the subpopulation with impaired baseline lung function (ppFEV1 <80%), while the normal baseline lung function subpopulation (ppFEV1 ≥80 %) showed hardly any improvement, probably due to a ceiling effect.

### FEF25-75

FEF25-75 (Forced Expiratory Flow 25-75%) refers to a fraction of the FVC (forced vital capacity) which is the maximal air volume which a patient can expire after maximal inspiration. FEF25-75 is the fraction of the FVC which is exhaled in the time span between exhalation of 25% of the FVC and exhalation of 75% of the FVC, divided by the time in which this volume is exhaled. Impairment in FEF25-75 can be indicative of obstruction of the small airways. In this study, FEF25-75 was determined by spirometry as described above.

Fig. 9 shows FEF25-75 for the whole patient population (left), as well as for the subpopulations (right) with normal baseline lung function (ppFEV1 ≥80 %) and impaired baseline lung function (ppFEV1 <80%) as explained above for the FEV1 results of Fig. 8. SAR443765 improved the FEF25-75 in the whole patient population (left panel), especially for D8 and D15. The results for the subpopulations (right panel) indicate that this overall improvement of FEF25-75 seen in the left panel is mainly due to the SAR443765 subpopulation with impaired baseline lung function (ppFEV1 <80%), which shows an improvement of around 0.4-0.5 L/s, while the normal baseline lung function subpopulation (ppFEV1 ≥80 %) showed hardly any improvement compared to baseline. This result is evidence that SAR443765 treatment reduces obstruction of the small airways in asthma patients.

### R5-20

As noted above, R5-20 is the difference between the respiratory resistance at 5 Hz and the respiratory resistance at 20 Hz. The resistance at 5 Hz indicates the resistance of the whole respiratory system (small and large airways) while the resistance at 20 Hz indicates the resistance within the large airways. Accordingly, an elevated R5-20 indicates increased resistance (and thus obstruction) of the small airways. R5-20 was determined by impulse oscillometry as described above.

Fig. 10 shows R5-20 for the whole patient population (left), as well as for the subpopulations (right) with normal baseline lung function (ppFEV1 ≥80 %) and impaired baseline lung function (ppFEV1 <80%) as explained above for the FEV1 results of Fig. 8. R5-20 was reduced in the whole patient population, the reduction exceeded the value of 0.31 cmH2O^{∗}s/L (0.03 kPa*s/L) which is regarded as the clinically meaningful threshold (Foy B, et al. Am J Respir Crit Care. 2019;200(8):982-991). The effect was most pronounced at D8 and D15. While there was virtually no effect of SAR443765 in the normal baseline lung function subpopulation (ppFEV1 ≥80 %), there was a remarkable reduction of R5-20 in the impaired baseline lung function subpopulation (ppFEV1 <80%). This indicates that the SAR443765 treatment reduces obstruction of the small airways especially in asthma patients with impaired lung function.

As noted above, area of reactance (AX) is calculated from the reactance measurement of the lung (see also Desiraju and Agrawal, 2016). It includes the total area dominated by the capacitance and reflects the capacitance (recoil) properties of the lung. As seen with reactance and fres, AX also increases in any disease of lung periphery. AX was determined by impulse oscillometry as described above.

Fig. 11 shows AX for the whole patient population (left), as well as for the subpopulations (right) with normal baseline lung function (ppFEV1 ≥80 %) and impaired baseline lung function (ppFEV1 <80%) as explained above for the FEV1 results of Fig. 8. SAR443765 reduced AX in the whole patient population, the reduction exceeded the value of 6.65 cmH2O/L (0.65 kPa/L) which is regarded as the clinically meaningful threshold (Abdo et al. Eur Respir J. 2023;61(5): 2201793). The effect was most pronounced at D8 and D15. While there was virtually no effect in the normal baseline lung function subpopulation (ppFEV1 ≥80 %), there was a remarkable reduction in the impaired baseline lung function subpopulation (ppFEV1 <80%) by SAR443765 administration. This indicates that the SAR443765 treatment reduces obstruction of the small airways especially in asthma patients with impaired lung function.

### Further biomarkers

In addition to the parameters shown above, the following biomarkers were determined in the above clinical trial with SAR443765: IL-5 level in serum; CCL26 (eotaxin-3) level in plasma; TARC (CCL17) level in serum; and IgE level in serum. The determination of those biomarker levels was performed according to methods commonly known in the field. The results at D29 are shown in Fig. 12. Treatment with SAR443765 led to a reduction from baseline for all observed biomarkers.

### Transcriptomic analysis

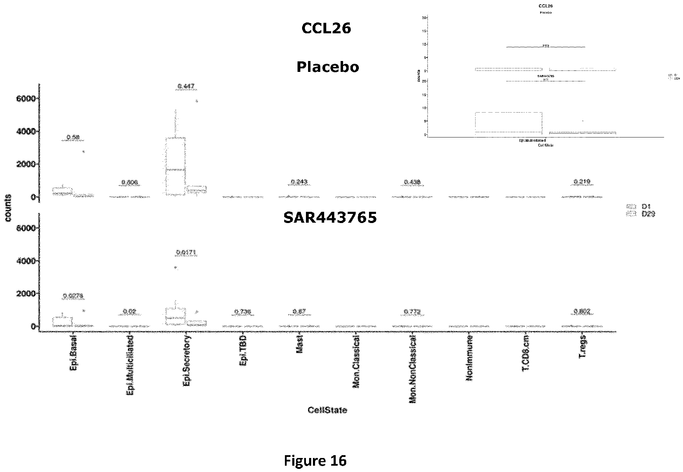

Transcriptomic analysis was performed by single cell RNA sequencing (scRNAseq). scRNAseq was used to determine the gene expression on single cell level in nasal brushing samples (NB) as well as peripheral blood leukocyte (PBL) samples. Samples were taken at D1 (baseline, before administration of SAR443765 or placebo) and D29. The analysis of gene expression allowed the identification of cell types and their proportion (Fig. 13, 14), correlation of change of this proportion with change in FeNO levels (Fig. 15), as well as the analysis of the expression of single genes per cell type (Fig. 16).

Fig. 13 depicts the different cell types found in nasal brushing samples. The change (D29 compared to D1) in proportion of each cell type (indicated as log2 of fold-change (FC)) on the x-axis is plotted against the p-value for this change (indicates as -log10 of the p-value) on the y-axis. In this analysis, p values ≤ 0.05 (corresponding to a value of at least around 1.3 on the y-axis) were considered as significant. It was found that in the SAR443765 group, the proportion of non-classical monocytes was significantly reduced while there was on significant change in the placebo group. As nasal brushing samples can be regarded as indicative for the whole respiratory tract, this is a first indication that the proportion of non-classical monocytes might be also reduced in the lung airways. The reduction of airway inflammation by SAR443765 might include the reduction of proportion of non-classical monocytes in the airways.

There was no significant change in the SAR443765 group or the placebo group for the other identified immune cell types.

Fig. 14 depicts the different cell types found in peripheral blood leukocyte (PBL) samples. The change (D29 compared to D1) in proportion of each cell type (indicated as log2 of fold-change (FC)) on the x-axis is plotted against the p-value for this change (indicates as -log10 of the p-value) on the y-axis. In this analysis, p values <_ 0.05 (corresponding to a value of at least 1.3 on the y-axis) were considered as significant. It was found that in the SAR443765 group, the proportion of NK cells as well as the proportion of CD8 T effector memory (em) cells was significantly reduced. The proportion of neutrophils was significantly increased, while additional measurements indicated that the total number of neutrophils did not increase. This is evidence for a rebalancing of the immune system by reduced type 2 inflammation and thus a reduced number of cells linked to type 2 inflammation. There were no significant changes in the placebo group. This indicates that the anti-inflammatory effect of SAR443765 might include the reduction of NK cell and CD8 T em cell proportion.

There was no significant change in the SAR443765 group or the placebo group for the other identified immune cell types.

Fig. 15 depicts the correlation of the change (D29 compared to D1) in FeNO levels and the change (D29 compared to D1) in proportion of NK cells in PBL samples. The y-axis shows the change in proportion of NK cells (log10 fold-change (FC) of NK cells) while the x-axis shows the change in FeNO levels in bbp. Each dot indicates the value for one participant. The left panel shows the SAR443675 group and the placebo group, while the right panel shows only the SAR443765. It is apparent that the correlation between change in FeNO level and change in NK cell proportion is much better for the SAR443765 group only, compared to the SAR443765 group combined with the placebo group. This suggests that that there might be a link between SAR443765-induced FeNO reduction and a reduction of NK cell proportion in blood, which could be a part of the therapeutic effect of SAR443765.

Fig. 16 shows the change (D29 compared to D1) in expression of the CCL26 gene for different cell types in the nasal brushing samples. While CCL26 expression was not significantly reduced the placebo group (upper panel), it was significantly reduced in basal epithelial cells, multiciliated epithelial cells and secretory epithelial cells after SAR443765 treatment (lower panel). The result for the multiciliated epithelial cells shown again in enlarged manner in the upper right corner of the figure. Those results corroborate the finding for CCL26/Eotaxin-3 on protein level (see Fig. 12).

### References:

Ziegler & Artis, Nat Rev Immunol (2010) 11:289-93
Gieseck III et al., Nat Rev Immunol. 2018 Jan;18(1):62-76
WO2021116182 - POLYPEPTIDES COMPRISING IMMUNOGLOBULIN SINGLE VARIABLE DOMAINS TARGETING IL-13 AND TSLP
Hamers-Casterman et al., Nature. 1993 Jun 3;363(6428):446-8
Muyldermans, J Biotechnol. 2001 Jun;74(4):277-302
WO 94/04678 - IMMUNOGLOBULINS DEVOID OF LIGHT CHAINS
Davies and Riechmann, Protein Eng. 1996 Jun;9(6):531-7
Conrath et al., J Biol Chem. 2001 Mar 9;276(10):7346-50
WO 96/34103 - VARIABLE FRAGMENTS OF IMMUNOGLOBULINS - USE FOR THERAPEUTIC OR VETERINARY PURPOSES
WO 99/23221 - MULTIVALENT ANTIGEN-BINDING PROTEINS
Venkataramani et al., Biochem Biophys Res Commun. 2018 Sep 26;504(1):19-24
Ober et al., Int Immunol. 2001 Dec;13(12):1551-9
Jönsson et al., Ann Biol Clin (Paris). 1993;51(1):19-26
Jönsson et al., Biotechniques. 1991 Nov;11(5):620-7.
Johnsson et al., J Mol Recognit. 1995 Jan-Apr;8(1-2):125-31
Johnnson et al. Anal Biochem. 1991 Nov 1;198(2):268-77
Abdiche et al. Anal Biochem. 2008 Jun 15;377(2):209-17
Drake et al., Anal Biochem. 2004 May 1;328(1):35-43
Fraley et al., Bioanalysis. 2013 Jul;5(14):1765-74
Global Initiative for Asthma 2020 Guidelines: Global strategy for asthma management and prevention, 2020 [cited 2021 May 07]. Available from: URL:www.ginasthma.org.
American Thoracic Society (ATS)/European Respiratory Society (ERS) Guidelines, 2019 update: Graham et al., Am J Respir Crit Care Med. 2019 Oct 15;200(8):e70-e88
Gauvreau et al., N Engl J Med. 2014 May 29;370(22):2102-10
Corren et al., N Engl J Med. 2017 Sep 7;377(10):936-946
Menzies-Gow et al., N Engl J Med. 2021 May 13;384(19):1800-1809 Korenblat et al., Respir Med. 2018 Jan;134:143-149
Corren et al., N Engl J Med. 2011 Sep 22;365(12):1088-98
Panettieri et al., Lancet Respir Med. 2018 Jul;6(7):511-525
Panettieri et al., Immunotherapy. 2018 Mar 1;10(6):473-490
Castro et al., Lancet Respir Med. 2014 Nov;2(11):879-890
Wenzel et al., Lancet. 2016 Jul 2;388(10039):31-44
Wechsler et al., N Engl J Med. 2021 Oct 28;385(18):1656-1668
Russell et al., Lancet Respir Med. 2018 Jul;6(7):499-510
Wechsler et al., Lancet Respir Med. 2022 Jul;10(7):650-660
Oostveen et al., Eur Respir J. 2003;22(6):1026-41
Peters et al., Appl Physiol Nutr Metab. 2016;41(5):538-47
Pride, Thorax. 1992;47(4):317-20
Làndsér et al., Chest. 1982;81(5):586-91
Clément et al., Chest. 1983;83(2):215-20
Otis et al., J Appl Physiol. 1956;8(4):427-43
Goldman et al., Respir Physiol Neurobiol. 2005;148(1-2):179-94
Oostveen E, et al. Eur RespirJ. 2013;42(6):1513-23
McNulty and Usmani, Eur Clin Respir J, 2014;1:10.3402/ecrj.v1.25898
Stockley et al., Int J Chron Obstruct Pulmon Dis. 2017;12: 2343-2353
Foy B, et al. Am J Respir Crit Care. 2019;200(8):982-991
Desiraju and Agrawal, Lung India. 2016 Jul-Aug; 33(4): 410-416.
Abdo et al., 2023 Eur Respir J. 2023 May 5;61(5):2201793
Haque et al., Genome Med. 2017 Aug 18;9(1):75
Slovin et al., Methods Mol Biol. 2021;2284:343-365
Chamberlain et al., fortune Journals 2023 ; 6(3), 152-177
Villani et al., Science. 2017 Apr 21;356(6335)
Deprez et al. Am J Respir Crit Care Med. 2020 Dec 15;202(12):1636-1645
Legebeke et al., Front Cell Dev Biol. 2022 Jun 15;10:907511
Vieira Braga et al., Nat Med. 2019 Jul;25(7):1153-1163
Miskoff et al., Cureus. 2019 Jun; 11(6): e4864
Cameli et al., Int J Mol Sci. 2020 Sep; 21(17): 6187
Li et al., J Allergy Clin Immunol. 2021 Mar;147(3):941-954
Tomita et al., J Allergy Clin Immunol. 1995 Aug;96(2):230-8
Eguiluz-Gracia et al., Clin Exp Allergy. 2018 Dec;48(12):1631-1639

## Claims

1. A compound that binds IL-13 and TSLP for use in the treatment of a pulmonary disease, wherein said treatment reduces the level of fractional exhaled nitric oxide (FeNO) by at least 18 ppb compared to a control.

2. The compound of use according to claim 1, wherein the treatment reduces the level of FeNO by at least 20 ppb, at least 30 ppb, or by at least 40 ppb compared to a control.

3. The compound for use according to any of the previous claims, wherein the pulmonary disease is asthma.

4. The compound for use according to claim 3, wherein the asthma is high eosinophilic asthma.

5. The compound for use according to claim 3, wherein the asthma is low eosinophilic asthma.

6. The compound for use according to any of the previous claims, wherein the control is baseline or wherein the control is placebo.

7. The compound for use according to any of the previous claims, wherein said reduction of FeNO level occurs within 4 weeks after the administration of the compound, wherein optionally said reduction of FeNO level occurs within 2 weeks after the administration of the compound, wherein optionally said reduction of FeNO level occurs within 1 week after the administration of the compound.

8. The compound for use according to any of the previous claims, wherein the compound that binds IL-13 and TSLP is a polypeptide, such as an antibody or an antibody fragment.

9. The compound for use according to claim 8, wherein the polypeptide comprises or consists of at least four ISVDs, wherein two ISVDs specifically bind IL-13 and two ISVDs specifically bind TSLP, wherein each of said at least four ISVDs comprises three complementarity determining regions (CDR1 to CDR3, respectively), wherein the at least four ISVDs are optionally linked via one or more peptidic linkers, and wherein:
a first ISVD comprises:
a CDR1 that is the amino acid sequence of SEQ ID NO: 7, a CDR2 that is the amino acid sequence of SEQ ID NO: 12 and a CDR3 that is the amino acid sequence of SEQ ID NO: 17,
a second ISVD comprises:
a CDR1 that is the amino acid sequence of SEQ ID NO: 8, a CDR2 that is the amino acid sequence of SEQ ID NO: 13 and a CDR3 that is the amino acid sequence of SEQ ID NO: 18,
a third ISVD comprises:
a CDR1 that is the amino acid sequence of SEQ ID NO: 9, a CDR2 that is the amino acid sequence of SEQ ID NO: 14 and a CDR3 that is the amino acid sequence of SEQ ID NO: 19, and
a fourth ISVD comprises:
a CDR1 that is the amino acid sequence of SEQ ID NO: 11, a CDR2 that is the amino acid sequence of SEQ ID NO: 16 and a CDR3 that is the amino acid sequence of SEQ ID NO: 21.

10. The compound for use according to claim 8 or 9, wherein the polypeptide comprises or consists of the amino acid sequence of SEQ ID NO: 1.

11. A compound that binds IL-13 and TSLP for use in the treatment of a pulmonary disease, wherein said treatment reduces the eosinophil count in blood by at least 30% compared to a control.

12. The compound of use according to claim 11, wherein said reduction of the eosinophil count occurs within 4 weeks after the administration of the compound, wherein optionally said reduction of the eosinophil count occurs within 2 weeks after the administration of the compound, wherein optionally said reduction of the eosinophil count occurs within 1 week after the administration of the compound.

13. A compound that binds IL-13 and TSLP for use in the treatment of a pulmonary disease, wherein said treatment reduces airway inflammation.

14. The compound of use according to claim 13, wherein said reduction of airway inflammation is **characterized by** a reduction of FeNO by at least 18 ppb, a reduction of eosinophil count by at least 30% and/or an increase of the forced expiratory volume in one second (FEV1) by at least 0.07 L compared to a control.

15. The compound of use according to claim 14, wherein said reduction of airway inflammation occurs within 4 weeks after the administration of the compound, wherein optionally said reduction of airway inflammation occurs within 2 weeks after the administration of the compound, wherein optionally said reduction of airway inflammation occurs within 1 week after the administration of the compound.

16. A compound which binds TSLP and/or IL-13 for use in reducing the FeNO level in a subject, wherein the reduction of FeNO level prevents a loss of lung function.

17. The compound for use according to claim 16, wherein said reduction of FeNO level is a reduction by at least 18 ppb.

18. The compound for use according to claim 16 or 17, wherein said reduction of FeNO level is a reduction to a level of below 25 ppb.

19. The compound for use according to any of claims 16-18, wherein the subject has a baseline FeNO level of at least 50 ppb, at least 35 ppb, or at least 25 ppb.

20. The compound for use according to any of claims 16-19, wherein the compound binds TSLP and IL-13.

21. The compound for use according to any of claims 16-20, wherein said a loss of lung function is linked to asthma.
